(19) Europäisches Patentamt
European Patent Office
Office européen des brevets

(11) Veröffentlichungsnummer : **0 441 467 B1**

# (12) EUROPÄISCHE PATENTSCHRIFT

(45) Veröffentlichungstag der Patentschrift :
**01.06.94 Patentblatt 94/22**

(51) Int. Cl.$^5$ : **C07C 401/00**

(21) Anmeldenummer : **91250032.9**

(22) Anmeldetag : **06.02.91**

(54) **Seitenketten-homologe Vitamin-D-Derivate, Verfahren zu ihrer Herstellung, diese enthaltende pharmazeutische Präparate, sowie deren Verwendung als Arzneimittel.**

(30) Priorität : **06.02.90 DE 4003854**
**30.10.90 DE 4034730**

(43) Veröffentlichungstag der Anmeldung :
**14.08.91 Patentblatt 91/33**

(45) Bekanntmachung des Hinweises auf die Patenterteilung :
**01.06.94 Patentblatt 94/22**

(84) Benannte Vertragsstaaten :
**AT BE CH DE DK ES FR GB GR IT LI LU NL SE**

(56) Entgegenhaltungen :
**WO-A-85/05622**
**WO-A-87/00834**
**US-A- 4 225 596**

(73) Patentinhaber : **SCHERING AKTIENGESELLSCHAFT**
**D-13342 Berlin (DE)**

(72) Erfinder : **Neef, Günter, Dr.**
**Markgraf-Albrecht-Strasse 19**
**W-1000 Berlin 31 (DE)**
Erfinder : **Kirsch, Gerald, Dr.**
**Luciusstrasse 6b**
**W-1000 Berlin 33 (DE)**
Erfinder : **Steinmeyer, Andreas, Dr.**
**Kaolingerplatz 3**
**W-1000 Berlin 19 (DE)**
Erfinder : **Schwarz, Katica**
**Zillestrasse 109**
**W-1000 Berlin 10 (DE)**
Erfinder : **Bräutigam, Matthias, Dr.**
**Droysenstrasse 8**
**W-1000 Berlin 12 (DE)**
Erfinder : **Thieroff-Ekerdt, Ruth, Dr.**
**Am Vierrutenberg 47**
**W-1000 Berlin 28 (DE)**
Erfinder : **Rach, Petra**
**Antwerpener Strasse 1**
**W-1000 Berlin 65 (DE)**

**EP 0 441 467 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft seitenketten-homologe Vitamin D-Derivate der Formel I

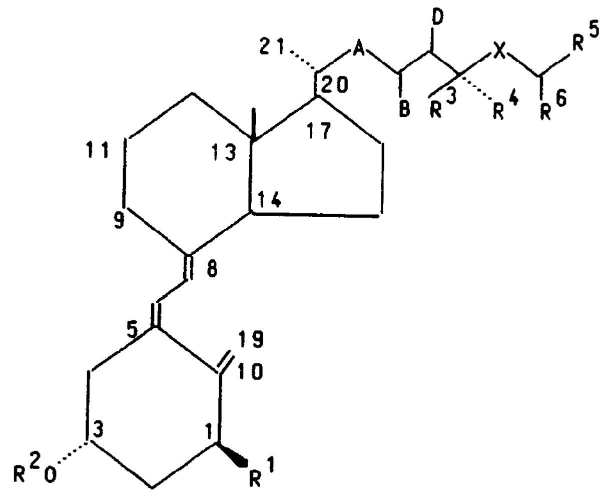

(I),

worin

| | |
|---|---|
| $R^1$ | ein Wasserstoffatom, eine Hydroxy- oder eine Acyloxygruppe mit 1 bis 9 Kohlenstoffatomen, |
| $R^2$ | ein Wasserstoffatom oder eine Acylgruppe mit 1 bis 9 Kohlenstoffatomen, |
| $R^3$ oder $R^4$ | eine Hydroxy- oder Acyloxygruppe mit 1 bis 9 Kohlenstoffatomen, und der jeweils andere Substituent ein Wasserstoffatom oder $R^3$ und $R^4$ gemeinsam ein Sauerstoffatom, |
| $R^5$ und $R^6$ | unabhängig voneinander jeweils einen linearen oder verzweigten Alkyl-rest mit bis zu 5 Kohlenstoffatomen, eine Trifluormethylgruppe oder gemeinsam einen mit dem tertiären Kohlenstoffatom gebildeten gesättigten, ungesättigten oder aromatischen carbocyclischen oder unter Einschluß von 1 oder 2 N-, O- oder S-Atomen heterocyclischen 3-, 4-, 5-oder 6-gliedrigen Ring, |
| B und D | entweder jeweils ein Wasserstoffatom oder gemeinsam eine zweite Bindung (E-konfigurierte Doppelbindung) und |
| | entweder |
| A | eine direkte Bindung zwischen den Kohlenstoffatomen 20 und 22 und |
| X | einen Alkylenoxyrest $-(CH_2)_nO-$ mit n = 1 bis 3 |
| | oder |
| A | eine Methylenbrücke ($-CH_2-$) zwischen den Kohlenstoffatomen 20 und 22 und |
| X | einen Alkylenrest $-(CH_2)_n-$ oder einen Alkylenoxyrest $-(CH_2)_nO-$ mit n = 1 bis 3, oder wenn A für eine direkte Bindung sowie B und D gemeinsam für eine zweite Bindung stehen, |

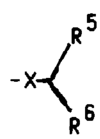

einen der Reste

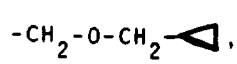

$-(CH_2)_2-\equiv$ oder $-(CH_2)_2-=$ bedeuten,

sowie ein Verfahren zu deren Herstellung, pharmazeutische Präparate, die diese Verbindungen enthalten sowie deren Verwendung zur Herstellung von Arzneimitteln.

Die für die Reste $R^1$, $R^2$ und innerhalb der Reste $R^3$ oder $R^4$ möglichen Acyloxy-bzw. Acylgruppen sind insbesondere von gesättigten Carbonsäuren oder auch der Benzoesäure abgeleitet. Andere geeignete Acylreste in $R^1$, $R^2$, $R^3$, $R^4$ umfassen solche, die cyclisch, acyclisch, carbocyclisch oder heterocyclisch - alle gegebenenfalls auch ungesättigt - sind. Die bevorzugten Reste leiten sich von $C_1$- bis $C_9$- , vorzugsweise $C_2$- bis $C_5$-, Alkancarbonsäuren ab, wie beispielsweise Acetyl-, Propionyl-, Butyryl-.

Bilden $R^5$ und $R^6$ gemeinsam mit dem tertiären Kohlenstoffatom einen gesättigten carbocyclischen Ring, so ist insbesondere an den Cyclopropyl-oder Cyclohexylring gedacht. Als Alkylgruppen für $R^5$ und $R^6$ kommen insbesondere solche mit 1 bis 5 Kohlenstoffatomen inbetracht, die geradkettig oder verzweigt sein können. Beispielhaft seien die Methyl-, Ethyl-, Propyl-sowie t-Butylgruppe genannt.

Bevorzugt gemäß vorliegender Erfindung sind seitenketten-homologe Vitamin-D-Derivate der allgemeinen Formel I, in welchen

$R^1$, $R^3$ oder $R^4$ für eine Hydroxygruppe oder

$R^5$ und $R^6$ für eine Methylgruppe oder gemeinsam mit dem tertiären Kohlenstoffatom für einen Cyclopropylring,

$R^2$ für ein Wasserstoffatom

steht/stehen und n 1 oder 2 ist.

Zwischen den Kohlenstoffatomen 22 und 23 (wenn A eine direkte Bindung bedeutet) oder zwischen den Kohlenstoffatomen 23 und 24 (wenn A eine Methylengruppe bedeutet) befindet sich vorzugsweise eine Doppelbindung. Besonders bevorzugt sind die Verbindungen

24-(1(R)-Hydroxy-4-methylpentyl)-9,10-secochola-5Z,7E,10(19),23E-tetraen-1(S),3(R)-diol,
24-(1(S)-Hydroxy-4-methylpentyl)-9,10-secochola-5Z,7E,10(19),23E-tetraen-1(S),3(R)-diol,
24-(1(R)-Hydroxy-3-methylbutyl)-9,10-secochola-5Z,7E,10(19),23E-tetraen-1(S),3(R)-diol,
24-(1(S)-Hydroxy-3-methylbutyl)-9,10-secochola-5Z,7E,10(19),23E-tetraen-1(S),3(R)-diol,
24-(1(R)-Hydroxy-3-methylbutyl)-9,10-secochola-5Z,7E,10(19)-trien-1(S),3(R)-diol,
24-(1(S)-Hydroxy-3-methylbutyl)-9,10-secochola-5Z,7E,10(19)-trien-1(S),3(R)-diol,
24-(1(R)-Hydroxy-3-isopropoxypropyl)-9,10-secochola-5Z,7E,10(19),23E-tetrae en-1(S),3(R)-diol,
24-(1(S)-Hydroxy-3-isopropoxypropyl)-9,10-secochola-5Z,7E,10(19),23E-tetraen-1(S),3(R)-diol,
24-Isopropoxymethyl-9,10-secochola-5Z,7E,10(19),22E-tetraen-1(S),3(R),24(R)-triol,
24-Isopropoxymethyl-9,10-secochola-5Z,7E,10(19),22E-tetraen-1(S),3(R),24(S)-triol,
24-(2-Isopropoxyethyl)-9,10-secochola-5Z,7E,10(19),22E-tetraen-1(S),3(R),24(R)-triol,
24-(2-Isopropoxyethyl)-9,10-secochola-5Z,7E,10(19),22E-tetraen-1(S),3(R),24(S)-triol,
26,27-Cyclo-24a,24b-dihomo-9,10-secocholesta-5Z,7E,10(19),23E-tetraen-1(S), 3(R),24a(R)-triol
26,27-Cyclo-24a,24b-dihomo-9,10-secocholesta-5Z,7E,10,23E-tetraen-1(S), 3(R),24a(S)-triol

Die natürlichen Vitamine $D_2$ und $D_3$ (vgl. allgemeine Formel V) sind an sich biologisch inaktiv und werden erst nach Hydroxylierung in 25-Position in der Leber bzw. in 1-Position in der Niere in deren biologisch aktive Metaboliten umgewandelt. Die Wirkung der Vitamine $D_2$ und $D_3$ besteht in der Stabilisierung des Plasma-$Ca^{++}$- und Plasma-Phosphat-Spiegels; sie wirken einem Absinken des Plasma-$Ca^{++}$-Spiegel entgegen.

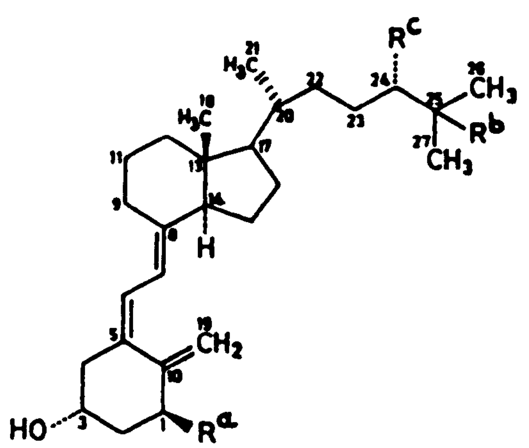

(V)

**Ergocalciferol:** $R^a = R^b = H$, $R^c = CH_3$, Vitamin $D_2$
**Doppelbindung C-22/23**
**Cholecalciferol:** $R^a = R^b = R^c = H$ Vitamin $D_3$
**25-Hydroxycholecalciferol:** $R^a = R^c = H$, $R^b = OH$
**1α-Hydroxycholecalciferol:** $R^a = OH$, $R^b = R^c = H$
**1α,25-Dihydroxycholecalciferol:** $R^a = R^b = OH$, $R^c = H$ Calcitriol

Neben ihrer ausgeprägten Wirkung auf den Calcium- und Phosphatstoffwechsel besitzen Vitamin $D_2$ und $D_3$ und seine synthetischen Abkömmlinge auch proliferationshemmende und zelldifferenzierende Wirkungen (H.F. De Luca, The Metabolism and Function of Vitamin D in Biochemistry of Steroid Hormones, Hrsg. H.L.J. Makin, 2nd Edition, Blackwell Scientific Publications 1984, S. 71-116).
Bei Vitamin D-Anwendung kann es aber zu Überdosierungserscheinungen kommen (Hypercalcämie).
In 24-Stellung hydroxylierte 1α-Cholecalciferole gehen bereits aus der DE-AS-25 26 981 hervor; sie besitzen eine geringere Toxizität als das entsprechende nicht-hydroxylierte 1α-Cholecalciferol. Die hydroxylierten Verbindungen zeigen eine selektive Aktivierung der intestinalen Calciumabsorption und eine schwächere Knochenabsorptionswirkung als 1α-Cholecalciferol. Die in der internationalen Patentanmeldung WO 87/00834 beschriebenen 24-Hydroxy-Vitamin-D-Analoga können für die Behandlung von durch abnormer Zellproliferation und/oder Zelldifferentiation hervorgerufenen Störungen beim Menschen und Tier dienen.

Für verschiedene 1,25-Dihydroxy-Homo-Vitamin-D-Derivate ist eine Dissoziation bezüglich der Eigenschaften Knochenabsorptionswirkung und HL-60 Zelldifferentiation schon kürzlich von De Luca erwähnt worden. Die Knochenabsorptionswirkung in vitro ist dabei ein direktes Maß für die Calciummobilisierung in vivo.

Es wurde nun gefunden, daß die erfindungsgemäßen seitenketten-homologen Vitamin-D-Derivate der allgemeinen Formel I im Vergleich zum Vitamin-D-Abkömmling Calcitriol (1α,25-Dihydroxycholecalciferol) überraschenderweise ein günstigeres Wirkungsspektrum aufweisen. Während die Effekte auf den Calcium- und Phosphatstoffwechsel deutlich abgeschwächt sind (Verringerung der Nebenwirkungen durch Überdosierung oder erforderlicher hoher Dosierung), bleiben die proliferationshemmenden und zelldifferenzierenden Wirkungen annähernd erhalten (Dissoziation).

Die Vitamin-D-Aktivität der erfindungsgemäßen Verbindungen wird mittels des Calcitriol-Rezeptortests bestimmt. Er wird unter Verwendung eines spezifischen Rezeptorproteins aus dem Darm rachitischer Hühner durchgeführt. Rezeptorhaltiges Bindungsprotein wird mit $^3$H-Calcitriol (0,5 ng/ml) in einem Reaktionsvolumen von 0,575 ml in Abwesenheit und in Anwesenheit der Prüfsubstanzen für eine Stunde in einem Teströhrchen inkubiert. Zur Trennung von freiem und rezeptorgebundenem Calcitriol wird eine Charcoal-Dextran-Absorption durchgeführt. Dazu werden 200 µl einer Charcoal-Dextran-Suspension jedem Teströhrchen zugeführt und bei 22°C für 30 Minuten inkubiert. Anschließend werden die Proben bei 1500 x g 10 Minuten bei 4°C zentrifugiert. Der Überstand wird dekantiert und nach ca. 1stündiger Äquilibrierung in Atom-Light in einem β-Zähler gemessen.
Die mit verschiedenen Konzentrationen der Prüfsubstanz sowie der Referenzsubstanz (unmarkiertes Calcitriol) bei konstanter Konzentration der Bezugssubstanz ($^3$H-Calcitriol) erhaltenen Kompetitionskurven werden in Beziehung zueinander gesetzt und ein Kompetitionsfaktor (KF) ermittelt.

Er ist definiert als Quotient aus den Konzentrationen der jeweiligen Prüfsubstanz und der Referenzsubstanz, die für 50%ige Kompetition erforderlich sind:

$$KF = \frac{\text{Konzentration Prüfsubstanz bei 50\% Kompetition}}{\text{Konzentration Referenzsubstanz bei 50\% Kompetition}}$$

Demnach besitzt
24-(1-Hydroxy-3-methylbutyl)-9,10-secochola-5Z,7E,10(19),23E-tetraen-1(S),3(R)-diol (Verbindung A) einen KF-Wert von 2,0 und
24-(1-Hydroxy-3-methylbutyl)-9,10-secochola-5Z,7E,10(19),23E-tetraen-1(S).3(S)diol (Verbindung B) einen KF-Wert von 3,6.

Zur Bestimmung der antiproliferativen Potenz der erfindungsgemäßen Verbindungen wird stellvertretend mit den Verbindungen A und B als Prüfsubstanzen der nachfolgend beschriebene Test durchgeführt:

Keratinocyten von neugeborenen Mäusen werden in Abwandlung der Methode von Yuspa, S. und Harris, C.C., "Altered differentiation of mouse epidermal cells treated with retinyl acetate in vitro", Exp. Cell Res. 86:95-105, 1974 präpariert und kultiviert.

Neonatale NMRI-Mäuse beiderlei Geschlechts werden durch Dekapitation getötet, die Haut abpräpariert, in einer Antibiotica-Antimykotika-Lösung gewaschen und mit der dermalen Seite nach unten in Dispase II-Lösung (1,2U/ml in Gewebekulturmedium M199 +25 mmol/l HEPES + 15% fötales Kälberserum (FCS) + 50 U/ml Penicillin/Streptomycin (P/S) (Präparationsmedium, PM) bei 4°C über Nacht inkubiert. Die Epidermis wird abgezogen und durch Trypsinierung eine Einzelzellsuspension hergestellt. Nach Zentrifugation wird das Zellsediment resuspendiert, nach Trypanblaufärbung die Zahl lebender kleiner runder Zellen bestimmt und die Zellen in einer Dichte von $4x10^5$ Zellen /cm$^2$ in Primaria-24-Loch-Platten in Gewebekulturmedium (M199 + 15% FCS + 50 U/ml P/S) ausgesät. Nach 24 Stunden Inkubation bei 37°C werden die Zellen mit phosphatgepufferter Salzlösung (PBS) gewaschen und weitere 24 Stunden in serumfreiem Gewebekulturmedium (M199 + 50 U/ml P/S + 0,5% Ethanol) mit und ohne Testsubstanzen bei 32,5°C inkubiert. Dann werden 0,4 µCi/50µl $^3$H-Methyl-thymidin (40Ci/mmol) zugegeben.Nach 4 Stunden wird das Medium abgesaugt und die Reaktion durch Zugabe von 500 µl eiskalter 10%iger Trichloressigsäure (TCA) beendet. Die Zellen werden

mit TCA und PBS gewaschen, durch Inkubation in einer Proteinase K-Lösung (10 mmol/l Tris-HCl, 10 mmol/l EDTA, 10 mmol/l NaCl, 0.2% Triton-X 100, pH 8,0, 50 μg/ml Proteinkinase K) lysiert und das Lysat durch Zentrifugation geklärt. Im Überstand wird szintillationsphotometrisch die Radioaktivität und, nach spezifischer Färbung der DNA mit Diamidinophenylindol (DAPI), die DNA-Konzentration fluoreszenzphotometrisch bestimmt.

Demnach hemmen Calcitriol sowie die Verbindungen A und B dosisabhängig den $^3H$-Thymidin-Einbau in DNA mit folgenden $IC_{50}$-Werten:

Calcitriol $2 \times 10^{-9}$ mol/l
Verbindung A $1 \times 10^{-8}$ mol/l
Verbindung B $3{,}2 \times 10^{-9}$ mol/l

Die differenzierungsstimulierenden Wirkungen von Calcitriol sowie den erfindungsgemäßen Verbindungen
26,27-Cyclo-24a,24b-dihomo-9,10-secocholesta-5Z,7E,10(19),23E-tetraen-1(S), 3(R),24a(R)-triol (Verbindung C) und
26,27-Cyclo-24a,24b-dihomo-9,10-secocholesta-5Z,7E,10(19),23E-tetraen-1(S), 3(R),24a(S)-triol (Verbindung D)
unterscheiden sich praktisch nicht.

Es ist literaturbekannt (Mangelsdorf, D. J. et al, J. Cell. Biol. 98: 391 - 398 (1984)), daß die Behandlung humaner Leukämiezellen (Promyelozytenzellinie HL 60) in vitro mit Calcitriol die Differenzierung der Zellen zu Makrophagen induziert.

Zur Quantifizierung der differenzierungsstimulierenden Wirkung von Calcitriolanaloga wird der nachfolgend aufgeführte Test durchgeführt:

HL 60-Zellen werden in Gewebekulturmedium (RPMI - 10% fetales Kälberserum bei 37°C in einer Atmosphäre 5 % $CO_2$ in Luft kultiviert.

Zur Substanztestung werden die Zellen abzentrifugiert und $2.8 \times 10^5$ Zellen/ml in phenolrotfreiem Gewebekulturmedium aufgenommen. Die Testsubstanzen werden in Ethanol gelöst und mit Gewebekulturmedium ohne Phenolrot auf die gewünschte Konzentration verdünnt. Die Verdünnungsstufen werden mit der Zellsuspension in einem Verhältnis von 1:10 gemischt und je 100 μl dieser mit Substanz versetzten Zellsuspension in eine Vertiefung einer 96-Loch-Platte pipettiert. Zur Kontrolle wird eine Zellsuspension analog mit dem Lösungsmittel versetzt.

Nach Inkubation über 96 Stunden bei 37°C in 5 % $CO_2$ in Luft wird in jede Vertiefung der 96-Loch-Platte zu der Zellsuspension 100 μl einer NBT-TPA-Lösung (Nitroblautetrazolium (NBT), Endkonzentration im Ansatz 1 mg/ml, Tetradecanoylphorbolmyristat-13-acetat (TPA), Endkonzentration im Ansatz $2 \times 10^{-7}$ mol/l) pipettiert.

Durch Inkubation über 2 Stunden bei 37°C und 5 % $CO_2$ in Luft wird infolge der intrazellulären Sauerstoffradikalfreisetzung, stimuliert durch TPA, in den zu Makrophagen differenzierten Zellen NBT zu unlöslichem Formazan reduziert.

Zur Beendigung der Reaktion werden die Vertiefungen der 96-Loch-Platte abgesaugt und die anhaftenden Zellen durch Zugabe von Methanol fixiert und nach Fixation getrocknet.

Zur Lösung der gebildeten intrazellulären Formazankristalle werden in jede Vertiefung 100μl Kaliumhydroxid (2 val/l) und 100 μl Dimethylsulfoxid pipettiert und 1 Minute ultrabeschallt. Die Konzentration von Formazan wird spektralphotometrisch bei 650 nm gemessen.

Als Maß für die Differenzierungsinduktion der HL 60-Zellen zu Makrophagen gilt die Konzentration an gebildetem Formazan. Die relative Wirksamkeit der Testsubstanz ergibt sich aus dem Quotienten $ED_{50}$ Testsubstanz / $ED_{50}$ Calcitriol.

Demnach besitzen Calcitriol, Verbindung C und Verbindung D die $ED_{50}$-Werte $1{,}8 \times 10^{-9}$ mol/l, $2{,}2 \times 10^{-9}$ mol/l bzw. $2{,}5 \times 10^{-9}$ mol/l.

Durch das verminderte Hypercalciämie-Risiko eignen sich die erfindungsgemäßen Substanzen in besonderer Weise zur Herstellung von Arzneimitteln für die Behandlung von Erkrankungen, die durch eine Hyperproliferation gekennzeichnet sind, z. B. hyperproliferative Erkrankungen der Haut (Psoriasis) und maligne Tumoren (Leukämie, Coloncarcinom, Mammacarcinom). In einer besonders bevorzugten Ausführungsform der Erfindung werden vor der Behandlung im Zielorgan Calcitriolrezeptoren nachgewiesen.

Die vorliegende Erfindung bezieht sich somit auch auf pharmazeutische Präparate, die mindestens eine Verbindung gemäß der allgemeinen Formel I zusammen mit einem pharmazeutisch verträglichen Träger enthalten. Die Verbindungen können formuliert werden als Lösungen in pharmazeutisch verträglichen Solventien oder als Emulsionen, Suspensionen oder Dispersionen in geeigneten pharmazeutischen Solventien oder Trägern oder als Pillen, Tabletten oder Kapseln, die in an sich bekannter Weise feste Trägerstoffe enthalten. Für eine topische Anwendung werden die Verbindungen vorteilhafterweise als Cremes oder Salben oder in einer ähnlichen, zur topischen Anwendung geeigneten Arzneimittelform formuliert. Jede derartige Formulierung kann auch andere pharmazeutisch verträgliche und nichttoxische Hilfsstoffe enthalten, wie z. B. Stabilisatoren, Antioxidantien, Bindemittel, Farbstoffe, Emulgatoren oder Geschmackskorrigentien. Die Verbindungen werden vorteilhafterweise durch Injektion oder intravenöse Infusion geeigneter steriler Lösungen oder als orale Dosierung über den Ernährungstrakt oder topisch on der Form von Cremes, Salben, Lotions oder geeigneter transdermaler Pflaster appliziert, wie in der EP-A-0387 077 beschrieben ist.

Die tägliche Dosis liegt bei

0,1 μg/Patient/Tag - 1000 μg (1 mg)/Patient/Tag,

vorzugsweise

1,0 μg/Patient/Tag - 500 μg/Patient/Tag.

Die erfindungsgemäßen Verbindungen werden im allgemeinen verabreicht analog zur Verabreichung des bekannten Mittels "Calcipotriol" zur Behandlung der Psoriasis.

Außerdem betrifft die Erfindung die Verwendung der Verbindungen gemäß Formel I zur Herstellung von Arzneimitteln.

Die Herstellung der seitenketten-homologen-Vitamin-D-Derivate der Formel I erfolgt erfindungsgemäß dadurch, daß eine Verbindung der allgemeinen Formel IV

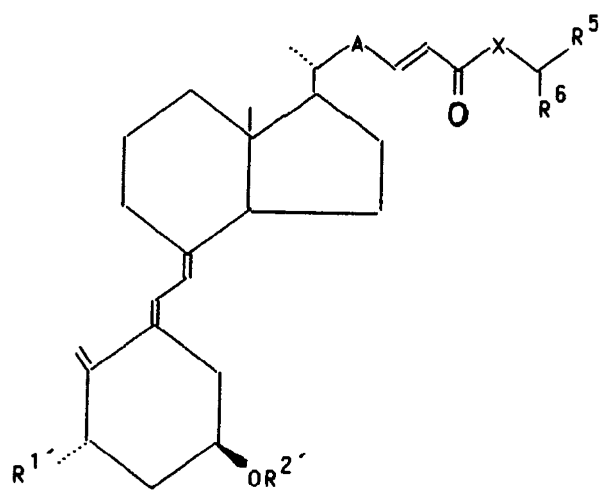

(IV),

worin

| | |
|---|---|
| $R^{1'}$ | ein Wasserstoffatom oder eine geschützte Hydroxygruppe und |
| $R^{2'}$ | eine Hydroxyschutzgruppe bedeuten und |
| A, X sowie $R^5$ und $R^6$ | die in Formel I angegebene Bedeutung haben, |

gewünschtenfalls nach selektiver Hydrierung der Doppelbindung in der Seitenkette zu einer Verbindung der allgemeinen Formel IVa

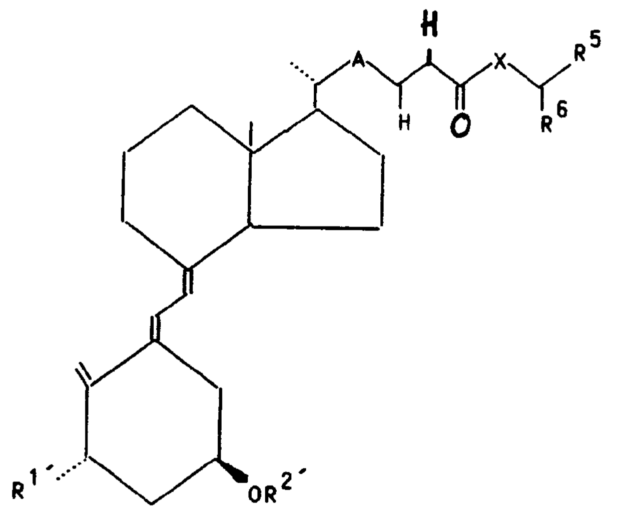

(IVa),

worin $R^{1'}$, $R^{2'}$, A, X sowie $R^5$ und $R^6$ die in Formel IV angegebene Bedeutung haben und gewünschtenfalls nach Reduktion der Carbonylfunktion und gegebenenfalls nach Trennung des Gemisches der durch die Reduktion gebildeten epimeren Hydroxyverbindungen der allgemeinen Formeln IIIa und IIIb

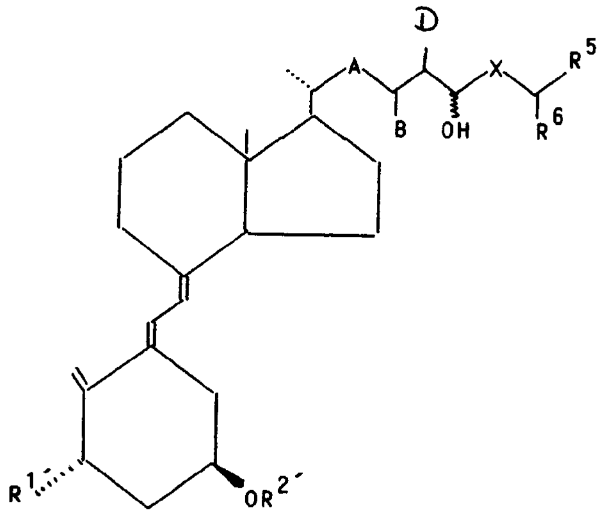

(IIIa) ~OH = α-OH
(IIIb) ~OH = β-OH

worin
$R^1$, $R^2$, A, X sowie $R^5$ und $R^6$ die in Formel IV und B und D die in Formel I angegebene Bedeutung haben, durch Bestrahlung mit ultraviolettem Licht unter Umkehr der Stereoisomerie an der 5,6-Doppelbindung in eine Verbindung der allgemeinen Formel II

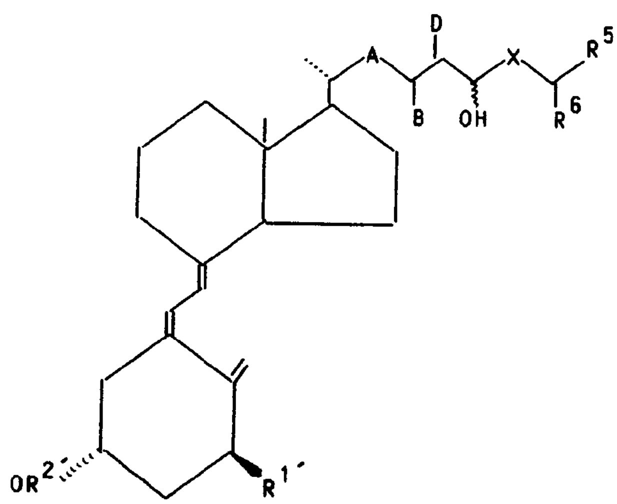

(II),

worin
$R^{1'}$, $R^{2'}$, A, B, D, X sowie $R^5$ und $R^6$ die in Formel IIIa/IIIb angegebene Bedeutung haben,
umgewandelt
und diese anschließend durch Abspaltung vorhandener Hydroxyschutzgruppen und gegebenenfalls durch partielle oder vollständige Veresterung der Hydroxygruppen in eine Verbindung der allgemeinen Formel I überführt wird.

Die Reduktion der Seitenketten-Carbonylfunktion in der Verbindung der allgemeinen Formel IV erfolgt beispielsweise mit Cer(III)chlorid/Natriumborhydrid in einem polaren Solvens. Bei der Reduktion entsteht sowohl das R- als auch das S-Hydroxyisomere der allgemeinen Formel IIIa bzw. IIIb. Die beiden Isomeren lassen sich chromatographisch trennen.

Gewünschtenfalls kann vor Reduktion der Carbonylfunktion die Doppelbindung in der Seitenkette selektiv hydriert werden. Als Hydrierungsmittel ist u.a. Lithium-tri-tert.-butoxy-aluminiumhydrid in einem polaren Solvens geeignet.

Die nachfolgende Umwandlung einer Verbindung der allgemeinen Formel IIIa/IIIb in eine Verbindung der allgemeinen Formel II erfolgt z.B. durch Bestrahlung mit ultraviolettem Licht in Gegenwart eines sogenannten "Triplett sensibilisators". Im Rahmen der vorliegenden Erfindung wird hierfür Anthracen verwendet. Durch Spaltung der pi-Bindung der 5,6-Doppelbindung, Rotation des A-Ringes um 180° um die 5,6-Einfachbindung und Reetablierung der 5,6-Doppelbindung wird die Stereoisomerie an der 5,6-Doppelbindung umgekehrt.

Anschließend werden vorhandene Hydroxyschutzgruppen abgespalten, vorzugsweise unter Verwendung von Tetra-n-butyl-ammoniumfluorid sowie gewünschtenfalls die freien Hydroxygruppen nach gängigen Verfahren partiell oder vollständig mit dem entsprechenden Carbonsäurehalogenid (Halogenid = Chlorid, Bromid) oder Carbonsäureanhydrid verestert.

**Herstellung der Ausgangsmaterialien**

1. 1(S),3(R)-Bis-(tert.-butyldimethylsilyloxy)-20(S)-formyl-9,10-secopregna-5E,7E,10(19)-trien 1:

Die Herstellung von 1 erfolgt nach M.J. Calverley, Tetrahydron 43, 4609 (1987); siehe auch internationale Patentanmeldung WO 87/00834. Dort ist auch die Herstellung der Ausgangsverbindung, worin $R^{1'}$ ein Wasserstoffatom ist, beschrieben.

2. 1(S),3(R)-Bis-(tert.-butyldimethylsilyloxy)-20(R)-methyl-9,10-secopregna-5E,7E,10(19)-trien-21-carbaldehyd 2

Der Aldehyd 2 wird nach einem neuen Verfahren hergestellt.

a. Zu einer Suspension von 1,8 g Natriumhydrid (80% in Öl) in 70 ml abs. THF tropft man bei 25°C eine Lösung von 15,57 g Diethylphosphonoethoxyessigsäureethylester (hergestellt nach W. Grell und H. Machleidt, Liebigs Ann. Chem. 699, 53 (1966)) in 200 ml THF. Nach Zugabe rührt man weitere 90 Minuten bei 60°C, kühlt erneut auf 25°C und gibt tropfenweise eine Lösung von 6,2 g 1 in 70 ml THF hinzu. Man rührt 2 Stunden unter Rückfluß, gießt die abgekühlte Reaktionslösung dann in Wasser und extrahiert mit Ethylacetat. Nach Trocknen ($Na_2SO_4$) und Einengen chromatographiert man das erhaltene Rohprodukt an Kieselgel mit Hexan/Ethylacetat. Die Hauptfraktion ergibt 5,2 g 1(S),3(R)-Bis-(tert-.-butyldimethylsilyloxy)-23-(ethoxy-9,10 secochola-5E,7E,10(19)-tetraen24-säure-ethylester als öliges Gemisch der C-22-Doppelbindungsisomeren.

b. 5,2 g des unter a. erhaltenen Produkts werden in 120 ml Toluol gelöst und bei 0°C langsam mit 20 ml einer 20%igen Lösung von Diisobutylaluminiumhydrid in Toluol versetzt. Nach 30 Minuten bei 0°C gießt man die Reaktionslösung vorsichtig in $NH_4Cl$-Lösung und extrahiert mit Ethylacetat. Nach üblicher Aufarbeitung erhält man 4,88 g 1(S),3(R)-Bis-(tert.-butyldimethylsilyloxy)-23-ethoxy-9,10-secochola-5E,7E,10(19),22-tetraen-24-ol als farbloses öliges Isomerengemisch, das ohne weitere Reinigung in die Folgestufe eingesetzt wird.

c. Die unter b. hergestellte Verbindung (4,88 g) wird in einem Gemisch aus 55 ml Dichlormethan und 55 ml 70%iger wäßriger Essigsäure 4 Stunden bei Raumtemperatur gerührt. Man neutralisiert anschließend durch Zugabe von $NH_3$-Lösung und extrahiert mit Dichlormethan. Das Rohprodukt wird an Kieselgel mit Hexan/Ethylacetat chromatographiert.. Auf diese Weise erhält man 2,02 g 1(S),3(R)-Bis-(tert.-butyldimethylsilyloxy)-24-hydroxy-9,10-secochola-5E,7E,10(19)-trien-23-on 5 als farbloses Öl. $^1H$-NMR ($CDCl_3$): δ=0,01 ppm (s,12H,Si-$CH_3$), 0,52 (s,3H,H-18), 0,81 und 0,84 (s, je 9H,Si-t-butyl), 0,90 (d,J=7Hz,3H,H-21 ), 3,09 (t,J=5Hz,1H,OH), 4,10 (dd,1H,H-24), 4,16 (m,1H,H-3), 4,21 (dd,1H,H-24), 4,39 (m,1H,H-1,) 4,88, 4,93 (s,je 1H,H-19), 5,77 6,39 (d,J=11Hz, je 1H,H-6,H-7).

d. Das unter c. erhaltene Produkt (2,02 g) wird in 25 ml Methanol und 25 ml THF gelöst und bei 0°C mit 300 mg Natriumborhydrid versetzt. Man rührt 1,5 Stunden bei 0°C, gießt das Reaktionsgemisch dann in $NH_4Cl$-Lösung und extrahiert mit Ethylacetat. Man erhält 1,75 g 1(S),3(R)-Bis-(tert.-butyldimethylsilyloxy)-9,10-secochola-5E,7E,10(19)-trien-23,24-diol 6 als farbloses, öliges Gemisch der 23-Epimeren, das als solches in die Folgereaktion eingesetzt wird.

e. Man löst 1,75 g des unter d. erhaltenen Produkts in 40 ml Toluol und gibt unter Eiswasserkühlung 1,23 g Bleitetraacetat portionsweise hinzu. Man rührt 30 Minuten, gibt erneut 1,0 g $Pb(OAc)_4$ hinzu und rührt weitere 15 Minuten bei +5 bis +10°C.

Zur Aufarbeitung versetzt man mit $NaHCO_3$-Lösung, filtriert die entstandene Suspension über Celite und extrahiert das Filtrat mit Ethylacetat. Das Rohprodukt wird an Kieselgel mit Hexan/Ethylacetat chromatographiert. Nach Kristallisation der Hauptfraktion aus Ethanol erhält man 560 mg 1(S),3(R)-Bis-(tert.-butyldimethylsilyloxy)-20(R)-methyl-9,10-secopregna-5E,7E,10(19)-trien-21-carbaldehyd vom Schmelzpunkt 101-104°C. Die Umsetzung des Aldehyds 1 bzw 2 mit einem Phosphoran der Formel

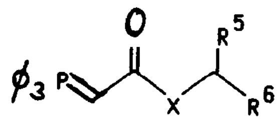

führt zu den Verbindungen der allgemeinen Formel IV (Wittig-Reaktion).

Herstellung der verwendeten Phosphor-Ylide:

1. Isobutylcarbonylmethylentriphenylphosphoran

a. Brommethylisobutylketon

50 ml Isobutylmethylketon in 240 ml Methanol werden bei 0°C mit 20 ml Brom versetzt und nach Zugabe noch 1,5 Stunden bei +10°C gerührt. Danach setzt man 360 ml Wasser zu und rührt weitere 16 Stunden bei Raumtemperatur.

Zur Aufarbeitung wird die Reaktionsmischung mit gesättigter Kochsalzlösung versetzt, die sich abscheidende organische Phase abgetrennt und die wäßrige Phase mit Ether extrahiert. Die vereinigten organischen Phasen werden mit 10%iger $Na_2CO_3$-Lösung gewaschen und über $Na_2SO_4$ getrocknet. Nach Filtration wird das Lösungsmittel im Wasserstrahlvakuum abgezogen und der Rückstand destilliert. Die Hauptfraktion enthält 53,7 g Brommethylisobutylketon vom $K_p^{15-20}$ 67-69°C.

b. Isobutylcarbonylmethyltriphenylphosphoniumbromid

Brommethylisobutylketon (53,6 g) und Triphenylphosphin (78,5 g) werden in einem 500 ml-Rundkolben innig vermischt und nach Abklingen der anfänglichen starken Wärmetönung 12 Stunden unter Stickstoff bei Raumtemperatur belassen. Danach wird die feste Reaktionsmasse in 330 ml Methylenchlorid aufgenommen und 30 Minuten unter Rückfluß erhitzt. Nach Zusatz von 500 ml Ether läßt man auf Raumtemperatur abkühlen und isoliert das Produkt durch Filtration. Nach Trocknung erhält man 111,7 g des Phosphoniumsalzes vom Schmelzpunkt 244-245°C.

c. Isobutylcarbonylmethylentriphenylphosphoran

111,6 g des unter b. erhaltenen Phosphoniumbromids werden sukzessive mit 1500 ml Methylenchlorid und 1500 ml 2N-NaOH versetzt und 30 Minuten bei Raumtemperatur gerührt. Die organische Phase wird abgetrennt, mit Wasser gewaschen und über $Na_2SO_4$ getrocknet. Der nach Einengen erhaltene feste Rückstand wird aus tert.-Butylmethylether umkristallisiert und ergibt 72,2 g des Ylids vom Schmelzpunkt 120-121°C.

2. Isoamylcarbonylmethylentriphenylphosphoran

Die Bildung der Titelverbindung erfolgt in Analogie zu dem unter 1. beschriebenen Verfahren durch Bromierung von Isoamylmethylketon, Umsetzung des Bromids mit Triphenylphosphin zum Phosphoniumsalz und Bildung des Ylids mit 2N NaOH.

Aus 50,0 ml Isoamylmethylketon und 18,2 ml Brom erhält man nach destillativer Aufreinigung 54,68 g 1-Brom-5-methyl-hexan-2-on vom $K_p^{15-20}$ 80-86°C.

Aus 54,58 g des Bromids und 74,14 g Triphenylphosphin erhält man 91,6 g des Phosphoniumsalzes vom Schmelzpunkt 230-233°C.

Aus 91,6 g des Phosphoniumsalzes erhält man nach Behandlung mit NaOH und Umkristallisation des Rohprodukts aus Methylenchlorid/Ester 69,8 g der Titelverbindung vom Schmelzpunkt 64-67°C.

3. Isopropoxymethylcarbonylmethylentriphenylphosphoran

2,43 g Natrium werden in 150 ml Isopropanol gelöst. Nach Zugabe von 20,0 g Chlormethylcarbonylmethylentriphenylphosphoran (R.F. Hudson et al., J. Org. Chem. 28 2446, 1963), in 200 ml Isopropanol gelöst, erhitzt man 8 Stunden unter Rückfluß.

Die abgekühlte Reaktionsmischung wird in Kochsalzlösung gegossen und mit Ethylacetat extrahiert. Der nach Einengen erhaltene ölige Rückstand wird an Kieselgel mit Ethylacetat chromatographiert. Man erhält 9,53 g der Titelverbindung vom Schmelzpunkt 134°C.

4. (2-Isopropoxyethyl)-carbonylmethylentriphenylphosphoran

a. 1-Brom-4-isopropoxy-butan-2-on

Eine Lösung aus 68,2 g 4-Isopropoxy-2-butanon (F.B. Hasan et al., J. Biolog. Chem. 256 , 7781, 1981) in 315 ml Methanol wird bei 0°C tropfenweise mit 26,9 ml Brom versetzt und danach 1,5 Stunden bei +10°C gerührt. Anschließend tropft man 470 ml Wasser zur Reaktionslösung und rührt 16 Stunden bei Raumtemperatur. Zur Aufarbeitung gießt man in gesättigte Kochsalzlösung und extrahiert mit Ether. Destillation des Rohprodukts ergibt 78,07 g des Bromderivats vom $K_p^{15-20}$ 95°C.

b. 4-Isopropoxy-2-oxo-butyl-triphenylphosphoniumbromid

Aus 78,0 g des unter a. erhaltenen Bromids und 97,85 g Triphenylphosphin erhält man nach dem unter 1. beschriebenen Verfahren 133,35 g des Phosphoniumsalzes vom Schmelzpunkt 183°C.

c. (2-Isopropoxyethyl)-carbonylmethylentriphenylphosphoran

Das unter b. erhaltene Phosphoniumbromid (133,2 g) wird wie unter 1. beschrieben mit 2N-NaOH in Methylenchlorid behandelt. Nach Umkristallisation des Rohprodukts aus Ethylacetat erhält man 64,38 g der Titelverbindung vom Schmelzpunkt 97°C.

5. (1-Ethylpropoxymethyl)-carbonylmethylentriphenylphorphoran

Eine Lösung von 3,04 g Natrium in 100 ml 3-Pentanol wird mit 25,0 g Chlormethylcarbonylmethylentriphenylphosphoran analog zur Darstellung von Isopropoxymethylcarbonylmethylentriphenylphosphoran umgesetzt. Die Titelverbindung wird als kristallisiertes Öl vom Schmelzpunkt 66-70°C erhalten.

6. Cyclpropylmethoxymethylcarbonylmethylentriphenylphosphoran

Eine Lösung von 5,58 g Natrium in 25,0 g Cyclopropylmethanol und 200 ml Toluoil wird mit 30,0 g Chlormethylcarbonylmethylentriphenylphosphoran analog zur Darstellung von Isopropoxymethylcarbonylmethylentriphenyl-phosphoran umgesetzt. Die Titelverbindung wird als Feststoff vom Schmelzpunkt 121°C erhalten.

7. (3-Butinyl)carbonylmethylentriphenylphosphoran

20,0 g Methylcarbonylmethylentriphenylphosphoran werden in 628 ml Tetrahydrofuran gelöst und bei -78°C mit 41,3 ml Butyllithium (1,6 molare Lösung in Hexan) tropfenweise versetzt. Anschließend werden 5,0 ml Propargylbromid zugetropft. Die Reaktionsmischung wird nach Erwärmung auf Raumtemperatur auf Eis/Kochsalz-Lösung gegeben und das Gemisch mit Essigester extrahiert. Nach Trocknung der organischen Phase mit Natriumsulfat werden 23,4 g Feststoff erhalten. Säulenchromatographische Reinigung (Kieselgel/Essigester) ergeben 15,4 g der Titelverbindung vom Schmelzpunkt 135-136°C.

8. (3-Butenyl)-carbonylmethylentriphenylphosphoran

Durch Reaktion von 15,0 g Methylcarbonylmethylentriphenylphosphoran in 471 ml Tetrahydrofuran mit 31,0 ml Butyllithium und 4,28 ml Allylbromid analog zu 7. erhält man die Titelverbindung als kristallisiertes Öl vom Schmelzpunkt 92-93°C.

Durch Variation der für die Herstellung des Wittig-Reagenzes eingesetzten Keto-Komponente lassen sich in analoger Weise weitere Phosphorane, die mit dem Aldehyd 1 oder 2 analog wie nachstehend beschrieben zu weiteren Verbindungen der allgemeinen Formel IV umgesetzt werden können, gewinnen.

## BEISPIEL 1

Eine Lösung von 1,6 g 1(S),3(R)-Bis-(tert.-butyldimethylsilyloxy)-20(R)-methyl-9,10-secopregna-5E,7E-,10(19)-trien-21-carbaldehyd in 50 ml Toluol wird nach Zusatz von 3,02 g Isoamylcarbonylmethylentriphenylphosphoran 16 Stunden bei 80°C unter Argon gerührt. Anschließend wird das Lösungsmittel unter vermindertem Druck abgezogen und der Rückstand an Kieselgel mit Hexan/Ethyl acetat chromatographiert. Die Hauptfraktion ergibt 1,15 g [1(S),3(R)-Bis-(tert.-butyldimethylsilyloxy)-9,10-secochola-5E,7E,10(19),23(E)-tetraen-24-yl]-4-methyl-pentan-1-on als farbloses Öl.

$^1$H-NMR ($CDCl_3$): $\delta$ = 0,01 ppm (s,12H,Si-$CH_3$), 0,56 (s,3H,H-18), 0,87 (s,18H,Sit.-butyl); 0,88 (d,J=7Hz,6H,C-$(CH_3)_2$), 0,95 (d,J=7Hz,3H,H-21); 4,25 (m,1H,H-3); 4,55 (m,1H,H-1); 4,94 und 5,00 (s,je 1H,H-19); 5,82 und 6,46 (d,J=11Hz, je 1H,H-6,H-7); 6,10 (d,J=16Hz,1H,H-24); 6,80 (m, 1H,H-23).

## BEISPIEL 2

Man löst 572 mg Cer(III)-chlorid-Heptahydrat in 10 ml Methanol und gibt die nach Beispiel 1 hergestellte Verbindung (1,10 g) in 5 ml Methanol gelöst hinzu. Nach Zusatz von 61 mg Natriumborhydrid wird 30 Minuten bei 0°C gerührt. Zur Aufarbeitung gießt man in Wasser, extrahiert mit Dichlormethan, trocknet ($Na_2SO_4$) und engt ein. Das so erhaltene Gemisch der diastereomeren Alkohole wird durch Chromatographie an Kieselgel mit Hexan/Ethylacetat getrennt. Man erhält in der Elutionsreihenfolge 290 mg 1(S),3(R)-Bis-(tert.-butyldimethylsilyloxy)-24-(1-hydroxy-4-methylpentyl)-9,10-seco-5E,7E,10(19),23(E)-cholatetraen (Epimer A) und 120 mg Epimer B. Die Epimeren zeigen identische NMR-Spektren.

$^1$H-NMR ($CDCl_3$): $\delta$ = 0,01 ppm (s,12H,Si-$CH_3$), 0,49 (s,3H,H-18), 0,86 (s,18H, Si-t.-butyl); 0,86 (d,J=7Hz,6H,C-$(CH_3)_2$); 0,88 (d,J=7Hz,3H,H-21); 4,16 (m,1H,H-3); 4,48 (m,1H,H-1; 4,88 und 4,93 (s, je 1H,H-19); 5,40 (dd,J=15,5 u. 7Hz,1H,H-24); 5,55 (m,1H,H-23): 5,77 und 6,40 (d,J=11Hz, je 1H,H-6,H-7).

**BEISPIEL 3:**

Eine Lösung von 290 mg des unter Beispiel 2 erhaltenen Produkts (Epimer A) in 80 ml Toluol wird nach Zusatz von 44 mg Anthracen und 0,01 ml Triethylamin in einem Pyrex-Tauchreaktor mittels einer Quecksilberhochdrucklampe (Philips HPK 125) bestrahlt. Die Bestrahlungszeit beträgt 3,5 Minuten, die Durchmischung der Lösung wird durch Einleiten eines Stickstoffstroms gewährleistet. Nach Einengen und Chromatographie an Kieselgel mit Hexan/Ethyl acetat erhält man 241 mg 1(S),3(R)-Bis-(tert.-butyldimethylsilyloxy)-24-(1-hydroxy-4-methylpentyl)-9,10secochola-5Z,7E,10(19)23(E)-tetraen als farbloses Öl.
$[\alpha]_D^{20}$ + 49,6° ($CHCl_3$, c=0,425).

Analoge Behandlung von 120 mg des nach Beispiel 2 erhaltenen polaren Isomeren (Epimer B) ergibt 113 mg als farbloses Öl.
$[\alpha]_D^{20}$ + 41,4° ($CHCl_3$, c=0,285).

**BEISPIEL 4:**

Eine Lösung von 225 mg des nach Beispiel 3 aus dem Epimeren A erhaltenen Produkts in 5 ml THF wird nach Zusatz von 1,31 ml einer 1M-Lösung von Tetrabutylammoniumfluorid in THF 60 Minuten bei 60°C gerührt. Nach dem Abkühlen gießt man in gesättigte Kochsalzlösung und extrahiert mit Ethylacetat. Das Rohprodukt wird an Kieselgel mit Hexan/Ethylacetat chromatographiert und liefert 85 mg 24-(1-Hydroxy-4-methylpentyl)-9,10-secochola-5Z,7E,10(19),23E-tetraen-1(S),3(R)-diol als weißen Schaum.
$^1H$-NMR ($CDCl_3$): $\delta$ = 0,57 ppm (s,3H,H-18), 0,84 (d,J=7Hz,3H,H-21); 0,92 (d,J=7Hz 6H,C-$(CH_3)_2$; 4,03 (m,1H,H-25); 4,23 (m,1H,H-3); 4,43 (m,1H,H-1); 5,00 und 5,33 (s, je 1H,H-19); 5,45 (dd,J=15,5 u. 7Hz,1H,H-24); 5,60 (m,1H,H-23); 6,02 und 6,38 (d,J=1Hz, Je 11H,H-6,H-7).

Analoge Behandlung des nach Beispiel 3 aus dem Epimer B erhaltenen Produkts (95 mg) ergibt 35 mg des epimeren Triols als farbloses Öl. Die NMR-Spektren der Epimeren sind identisch.

**BEISPIEL 5**

In Analogie zu dem unter Beispiel 1 beschriebenen Verfahren setzt man 2,05 g 1(S),3(R)-Bis-(tert.-butyldimethylsilyloxy)-20-(R)-methyl-9,10-secopregna-5E,7E,10(19)-trien-21-carbaldehyd in 53 ml Toluol mit 3,4 g Isobutylcarbonylmethylentriphenylphosphoran um. Nach chromatographischer Reinigung erhält man [1(S),3(R)-Bis-(tert.-butyldimethylsilyloxy)-9,10-secochola-5E,7E10(19),23(E)-tetraen24-yl]-3-methyl-butan-1-on vom Schmelzpunkt 79-81°C (aus Ethanol),
$[\alpha]_D^{20}$ + 52,6° ($CHCl_3$, c=0,500).

**BEISPIEL 6**

Durch Reduktion von 1,75 g des unter Beispiel 5 erhaltenen Produkts unter den Bedingungen des Beispiels 2 erhält man 1(S),3(R)-Bis-(tert.-butyldimethylsilyloxy)-24-(1(R,S)-hydroxy-3-methylbutyl)-9,10-secochola-5E,7E,10(19),23E-tetraen als öliges Gemisch der Epimeren. Durch Chromatographie an Kieselgel mit Hexan/ Ethylacetat erhält man in der Elutionsreihenfolge 780 mg Epimer A und 600 mg Epimer B als farblose Öle, die NMR-Spektroskopisch nicht unterscheidbar sind.

**BEISPIEL 7**

Durch triplett-sensibilisierte Photoisimerisierung analog Beispiel 3 und anschließende Silyletherspaltung analog Beispiel 4 erhält man aus 700 mg des nach Beispiel 6 hergestellten Epimers A 240 mg 24-(1-Hydroxy-3-methyl-butyl)9,10-secochola-5Z,7E,10(19),23E-tetraen-1(S),3(R)-diol (Verbindung A) vom Zersetzungsintervall 119-125°C, $[\alpha]_D^{20}$ + 38,8° (Methanol, c=0,505).

Analoge Behandlung von 330 mg Epimer 8 ergibt 129 mg 24-(1-Hydroxy-3-methylbutyl)9,10-secochola-5Z,7E,10(19),23E-tetraen-1(S),3(S)-diol (Verbindung B) vom Zersetzungsintervall 139-145°C, $[\alpha]_D^{20}$ + 54,8° (Methanol, c=0,505).

**BEISPIEL 8**

Eine Lösung von 170 mg des nach Beispiel 5 erhaltenen Produkts in 5 ml THF wird nach Zusatz von 200 mg Lithium-tri-tert.-butoxy-aluminiumhydrid 90 Minuten bei Raumtemperatur gerührt. Zur Aufarbeitung versetzt man mit 0,8 ml gesättigter $NH_4Cl$-Lösung, filtriert und engt das Filtrat ein. Chromatographie des Rohprodukts an $Al_2O_3$ (Merck, neutral, Stufe III) ergibt 108 mg 1-[1(S),3(R)-Bis-(tert.-butyldimethylsilyloxy)-9,10-secochola-5E,7E,10(19)-trien-24-yl]-3-methyl-butan-1-on als farbloses Öl.

$^1$H-NMR ($CDCl_3$): $\delta$ = 0,53 ppm (s,3H,H-18); 4,22 (m,1H,H-3); 4,54 (m,1H,H-1); 4,93 und 4,98 (m, Je 1H,H-19); 5,82 und 6,46 (d,J=11Hz, je 1H,H-6,H-7).

**BEISPIEL 9**

Photochem. Doppelbindungsisomerisierung analog Beispiel 3 und Silyletherspaltung analog Beispiel 4 ergeben aus 100 mg des Produkts von Beispiel 8 50 mg 1-[1(S),3(R)-Dihydroxy-9,10-secochola-5Z,7E,10(19)-trien-24-yl]-3-methyl-butan1-on.

UV (Methanol): =212 nm ($\varepsilon$=14 300), 265 (15 860).

**BEISPIEL 10**

Umsetzung von 1,6 g 1(S),3(R)-Bis-(tert.-butyldimethylsilyloxy)-20(R)-methyl9,10-secopregna-5E,7E,10(19)-trien-21-carbaldehyd mit (2-Isopropoxyethyl)-carbonylmethylentriphenylphosphoran analog Beispiel 1 ergibt 1,15 g 1-[1(S),3(R)-Bis-(tert.-butyldimethylsilyloxy)-9,10-secochola-5E,7E,10(19)23(E)-tetraen-24-yl]-3-isopropoxy-propan-1-on als farbloses Öl.

$^1$H-NMR ($CDCl_3$): $\delta$ = 0,01 ppm (s,12H,Si-$CH_3$),0,55 (s,3H,H-18), 0,86 und 0,90 (s, je 9H,Si-t.-butyl); 0,96 (d,J=7Hz,3H,H-21); 1,15 (d,J=7Hz,6H,C($CH_3$)$_2$); 3,60 (m,1H,CH-0): 3,73 (t,J=7Hz,2H,$CH_2$-0); 4,23 (m,1H,H-3); 4,55 (m,1H,H-1); 4,95 und 5,00 (m, je 1H,H-19); 5,83 und 6,46 (d,J=11Hz, je 1H,H-6,H-7); 6,11 (d,J=15,5Hz,1H,H-24); 6,87 (m,1H,H-23).

**BEISPIEL 11**

Durch Reduktion analog Beispiel 2, Photoisomerisierung analog Beispiel 3 und Silyletherspaltung analog Beispiel 4 erhält man aus 1,05 g des nach Beispiel 10 dargestellten Produkts 143 mg 24-(1(R,S)-Hydroxy-3-isopropoxy-propyl)-9,10-secochola-5Z,7E,10(19),23-tetraen-1(S),3(R)-diol als 1:1-Gemisch der Diastereomeren, die durch Hochdruckflüssigkeitschromatographie getrennt werden. Die Isomeren weisen identische NMR-Spektren auf.

$^1$H-NMR ($CDCl_3$): $\delta$ =0,57 ppm (s,3H,H-18), 0,94 (d,J=7Hz,3H,H-21); 1,15 (d,J=7Hz, 6H,C($CH_3$)$_2$), 4,17 (m,1H,H-3); 4,21 (m,1H,H-25); 4,38 (m,1H,H-1); 4,98 und 5,29 (m, je 1H,H-19); 5,45 (dd, J=15,5 und 7Hz,1H,H-24); 5,63 (m,1H,H-23); 6,02 und 6,38 (d,J=11Hz, je 1H,H-6,H-7).

**BEISPIEL 12**

Ausgehend von Aldehyd 1 und Isopropoxymethylcarbonylmethylentriphenylphosphoran wird analog der Sequenz der Beispiele 1-4 Isomer B (5Z,7E,22E-1(S),3(R),24(S)-9,10-Seco-24a,24b-dihomo-24b-oxacholesta-5,7,10(19)22-tetraen-1,3,24-triol) vom Schmelzpunkt 131-132°C erhalten.

**BEISPIEL 13**

Ausgehend von Aldehyd 1 und (2-Isopropoxyethyl)-carbonylmethylentriphenylphosphoran wird analog der Sequenz der Beispiele 1-4 Isomer B (5Z,7E,22E-1(S),3(R),24(S)-9,10-Seco-24a,24b,24c-trihomo-24c-oxacholesta-5,7,10(19)22-tetraen-1,3,24-triol) vom Schmelzpunkt 125-126°C erhalten.

**BEISPIEL 14**

Analog zu Beispiel 1 setzt man 0,85 g 1(S),3(R)-Bis-(tert.-butyldimethylsilyloxy)-20(R)-methyl-9,10-secopregna-5E,7E,10(19)-trien-21-carbaldehyd mit 4,5 g Cyclopropylmethylcarbonyltriphenylphosphoran um. Nach chromatographischer Reinigung an Kieselgel mit Hexan/Essigester werden 500 mg 1(S), 3(R)-Bis-(tert.-butyldimethylsilyloxy)-26,27-cyclo-24a,24b-diohomo-9,10-secocholesta-5E,7E,10(19)-23E-tetraen-24a-on als farbloser Schaum erhalten.

$^1$H-NMR($CDCl_3$): $\delta$=0,01 ppm (s,12H, Si-$CH_3$); 0,09 und 0,50 (m,je 2H,H-26 u. H-27); 0,50(s,3H,H-18); 0,83 u. 0,85 (s, je 9H,Si-t.-butyl); 0,91 (d,J=7,3 Hz,3H, H-21); 0,96(m,1H,H-25) ; 2,47 (d,J=6 Hz,2H,H-24b); 4,16(m,1H,H-3); 4,47(m,1H,H-1) ; 4,89 u. 4,93(s,je1H,H-19); 5,77 u. 6,40(d,J=11 Hz,je1H,H-6 u. H-7); 6,08(d,J=15,5 Hz,H-24); 6,75(ddd,J=15,5, 9, 6,5 Hz,1H,H-23).

**BEISPIEL 15**

Reduktion des unter Beispiel 14 erhaltenen Produkts analog Beispiel 2 liefert 200 mg 1(S),3(R)-Bis-(tert.-butyldimethylsilyloxy)-26,27-cyclo-24a,-24b-dihomo-9,10-secocholesta-5E,7E,10(19),23E-tetraen-24a(R,S)-ol als öliges Gemisch der Epimeren, die NMR-spektroskopisch nicht unterscheidbar sind.

$^1$H-NMR($CDCl_3$): δ=0,01 ppm(s,12H,Si-$CH_3$); 0,09 u. 0,40(m,je2H,H-26 u. H-27); 0,50(s,3H,H-18); 0,68(m,1H,H-25); 0,81 u. 0,86(s,je9H,Si-t.-butyl); 0,88-(d,J=7 Hz,3H,H-21); 1,40(t,J=7 Hz,H-24b); 4,13(m,1H,H-24a); 4,17(m,1H,H-3); 4,49(m,1H,H-1); 4,88 u. 4,93(s,je 1H,H-19); 5,45(dd,J=15,5, 6,5 Hz,1H,H-24); 5,59(ddd, J=15,5, 7, 6,5 Hz,1H,H-23); 5,77 u. 6,40(d,J=11 Hz,je 1H,H-6 u. H-7).

**BEISPIEL 16**

Analog zu Beispiel 3 erhält man durch triplettsensibilisierte Photoisomerisierung und Abspaltung der Schutzgruppen analog Beispiel 4 aus 190 mg der unter Beispiel 15 beschriebenen Verbindung 86 mg 26,27-Cyclo-24a,24b-dihomo-9,10-secocholesta-5Z,7E,10(19), 23E-tetraen-1(S),3(R),24a(R,S)-triol als 1:1-Gemisch der Diastereomeren, die durch Hochdruckflüssigkeitschromatographie getrennt werden. Die NMR-Spektren beider Diastereomere sind identisch.

$^1$H-NMR($CDCl_3$): δ=0,09 u. 0,49(m,je 2H,H-26 u. H-27); 0,53(s,3H,H-18); 0,70(m,1H,H-25); 0,93(d,3=7 Hz,3H,H-21); 4,18(m,1H,H-24a); 4,22(m,1H,H-3); 4,43(m,1H, H-1); 5,00 u. 5,32(s,je1H,H-19); 5,50(dd,J=15,5, 6,5 Hz,H-24); 5,64(ddd,J=15,5, 7, 6,5 Hz,1H,H-23); 6,02 u. 6,38(d,J=11Hz,je1H,H-6 u. H-7).

**BEISPIEL 17**

Ausgehend vom Aldehyd 1 und (1-Ethylpropoxymethyl)-carbonylmethylentriphenylphosphoran wird analog der Sequenz der Beispiele 1-4 Isomer B (5Z,7E,22E -1(S),3(R),24(S)-26,27-Dimethyl-24a,24b-dihomo-24b-oxa-9, 10-secocholesta-5, 7,10(19)22-tetraen-1,3,24-triol) vom Schmelzpunkt 103-105°C erhalten.

**BEISPIEL 18**

Ausgehend von Aldehyd 1 und Cyclopropylmethoxymethylcarbonylmethylentriphenylphosphoran wird analog der Sequenz der Beispiele 1-4 Isomer B (5Z,7E,22E -1(S),3(R),24(S)-26,27-Cyclo-24a,24b,24c-trihomo-24b-oxa-9,10-secocholesta-5,7,10(19)22-tetraen-1,3,24-triol) erhalten.

$^1$H-NMR (DMSO-$d_6$) S=0,16 ppm(m,2H); 0,43(m,2H); 0,53(S,3H); 1,00(d,J=6Hz,3H); 3,21(m,4H); 4,00(m,2H); 4,19(m,1H); 4,51(d,J=5Hz,1H); 4,70(d,J=5Hz,1H); 4,75(m,1H); 4,82(d,J=5Hz,1H); 5,21(m,1H); 5,39(m,2H); 5,98(d,J=11Hz,1H); 6,18(d,J=11Hz,1H).

**BEISPIEL 19**

Ausgehend von Aldehyd 1 und (3-Butinyl)-carbonylmethylentriphenylphosphoran wird analog der Sequenz der Beispiele 1-4 Isomer B (5Z,7E,22E-1(S),3(R),24(S)-24-(3-Butinyl)-9,10-secochola-5,7,10(19)22-tetraen-1,3,24-triol) vom Schmelzpunkt 115-118°C erhalten.

**BEISPIEL 20**

Ausgehend von Aldehyd 1 und (3-Butenyl)-carbonylmethylentriphenylphosphoran wird analog der Sequenz der Beispiele 1-4 Isomer B (5Z,7E,22E--1(S),3(R),24(S)-24-(3-Butenyl)-9,10-secochola-5, 7,10(19)22-tetraen-1,3,24-triol) vom Schmelzpunkt 146-147°C erhalten.

## Patentansprüche

1. Seitenketten-homologe Vitamin D-Derivate der Formel I

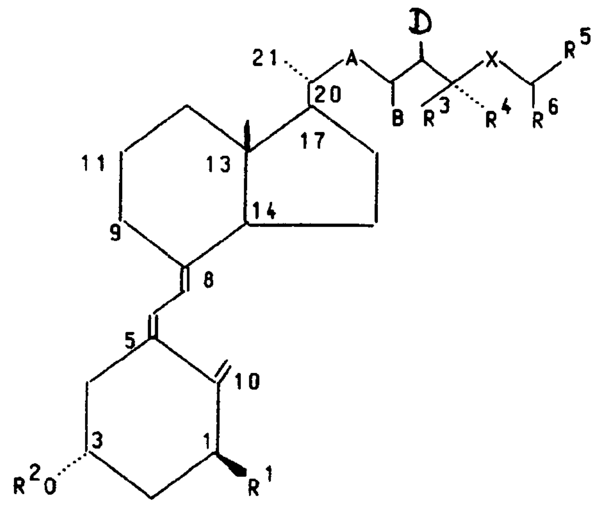

(I),

worin

| | |
|---|---|
| $R^1$ | ein Wasserstoffatom, eine Hydroxy- oder eine Acyloxygruppe mit 1 bis 9 Kohlenstoffatomen, |
| $R^2$ | ein Wasserstoffatom oder eine Acylgruppe mit 1 bis 9 Kohlenstoffatomen, |
| $R^3$ oder $R^4$ | eine Hydroxy- oder Acyloxygruppe mit 1 bis 9 Kohlenstoffatomen, und der Jeweils andere Substituent ein Wasserstoffatom oder $R^3$ und $R^4$ gemeinsam ein Sauerstoffatom, |
| $R^5$ und $R^6$ | unabhängig voneinander jeweils einen linearen oder verzweigten Alkyl-rest mit bis zu 5 Kohlenstoffatomen, eine Trifluormethylgruppe oder gemeinsam einen mit dem tertiären Kohlenstoffatom gebildeten gesättigten, ungesättigten oder aromatischen carbocyclischen oder unter Einschluß von 1 oder 2 N-, O- oder S-Atomen heterocyclischen 3-, 4-, 5-oder 6-gliedrigen Ring, |
| B und D | entweder jeweils ein Wasserstoffatom oder gemeinsam eine zweite Bindung (E-konfigurierte Doppelbindung) und |
| | entweder |
| A | eine direkte Bindung zwischen den Kohlenstoffatomen 20 und 22 und |
| X | einen Alkylenoxyrest $-(CH_2)_nO-$ mit n = 1 bis 3 |
| | oder |
| A | eine Methylenbrücke ($-CH_2-$) zwischen den Kohlenstoffatomen 20 und 22 und |
| X | einen Alkylenrest $-(CH_2)_n-$ oder einen Alkylenoxyrest $-(CH_2)_nO-$ mit n = 1 bis 3, oder wenn A für eine direkte Bindung sowie B und D gemeinsam für eine zweite Bindung stehen, |

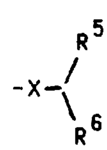

einen der Reste

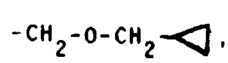

$-(CH_2)_2-\equiv$ oder $-(CH_2)_2-=$ bedeuten.

2. Vitamin-D-Derivate nach Anspruch 1, worin $R^1$ für eine Hydroxygruppe steht.

3. Vitamin-D-Derivate nach Anspruch 1, worin $R^2$ für ein Wasserstoffatom steht.

4. Vitamin-D-Derivate nach Anspruch 1, worin $R^3$ oder $R^4$ eine Hydroxygruppe bedeutet.

5. Vitamin-D-Derivate nach Anspruch 1, worin n in X 1 oder 2 ist.

6. Vitamin-D-Derivate nach Anspruch 1, worin $R^5$ und $R^6$ für Methylgruppen stehen.

7. Vitamin-D-Derivate nach Anspruch 1, worin $R^5$, $R^6$ und das tertiäre Kohlenstoffatom gemeinsam für einen Cyclopropylring stehen.

8. 24-(1(R)-Hydroxy-4-methylpentyl)-9,10-secochola-5Z,7E,10(19),23E-tetraen-1(S),3(R)-diol,
24-(1(S)-Hydroxy-4-methylpentyl)-9,10-secochola-5Z,7E,10(19),23E-tetraen-1(S),3(R)-diol,
24-(1(R)-Hydroxy-3-methylbutyl)-9,10-secochola-5Z,7E,10(19),23E-tetraen-1(S),3(R)-diol,
24-(1(S)-Hydroxy-3-methylbutyl)-9,10-secochola-5Z,7E,10(19),23E-tetraen-1(S),3(R)-diol,
24-(1(R)-Hydroxy-3-methylbutyl)-9,10-secochola-5Z,7E,10(19)-trien-1(S),3(R)-diol,
24-(1(S)-Hydroxy-3-methylbutyl)-9,10-secochola-5Z,7E,10(19)-trien-1(S),3(R)-diol,
24-(1(R)-Hydroxy-3-isopropoxypropyl)-9,10-secochola-5Z,7E,10(19),23E-tetraen-1(S),3(R)-diol,
24-(1(S)-Hydroxy-3-isopropoxypropyl)-9,10-secochola-5Z,7E,10(19),23E-tetraen-1(S),3(R)-diol,
24-Isopropoxymethyl-9,10-secochola-5Z,7E,10(19),22E-tetraen-1(S),3(R),24(R)-triol,
24-Isopropoxymethyl-9,10-secochola-5Z,7E,10(19),22E-tetraen-1(S),3(R),24(S)-triol,
24-(2-Isopropoxyethyl)-9,10-secochola-5Z,7E,10(19),22E-tetraen-1(S),3(R),24(R)triol,
24-(2-Isopropoxyethyl)-9,10-secochola-5Z,7E,10(19),22E-tetraen-1(S),3(R),24(S)triol,
26,27-Cyclo-24a,24b-dihomo-9,10-secocholesta-5Z,7E,10(19),23E-tetraen-1(S),3(R)24a(R)-triol,
26,27-Cyclo-24a,24b-dihomo-9,10-secocholesta-5Z,7E,10(19),23E-tetraen-1(S),3(R)24a(S)-triol

9. Verfahren zur Herstellung von seitenketten-homologen Vitamin-D-Derivaten der Formel I

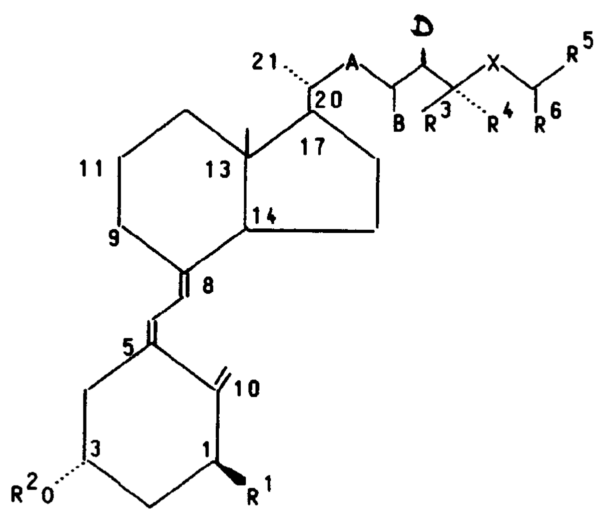

(I),

worin $R^1$,$R^2$, $R^3$, $R^4$, $R^5$ und $R^6$ sowie A, B, D und X die in Anspruch 1 angegebene Bedeutung haben, dadurch gekennzeichnet, daß eine Verbindung der allgemeinen Formel IV

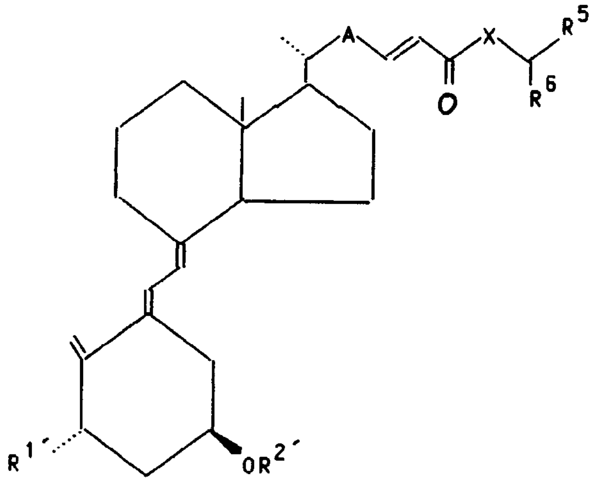

(IV),

worin

| | |
|---|---|
| $R^{1'}$ | ein Wasserstoffatom oder eine geschützte Hydroxygruppe und |
| $R^{2'}$ | eine Hydroxyschutzgruppe bedeuten und |
| A, X sowie $R^5$ und $R^6$ | die in Formel I angegebene Bedeutung haben, |

gewünschtenfalls nach selektiver Hydrierung der Doppelbindung in der Seitenkette zu einer Verbindung der allgemeinen Formel IVa

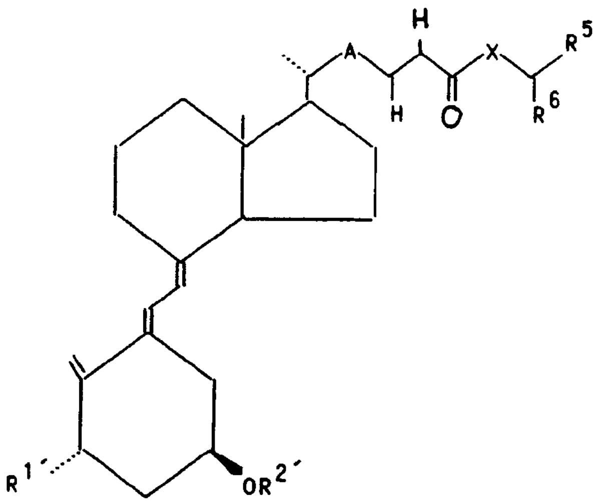

(IVa),

worin $R^{1'}$, $R^{2'}$, A, X sowie $R^5$ und $R^6$ die in Formel IV angegebene Bedeutung haben und gewünschtenfalls nach Reduktion der Carbonylfunktion und gegebenenfalls nach Trennung des Gemisches der durch die Reduktion gebildeten epimeren Hydroxyverbindungen der allgemeinen Formeln IIIa und IIIb

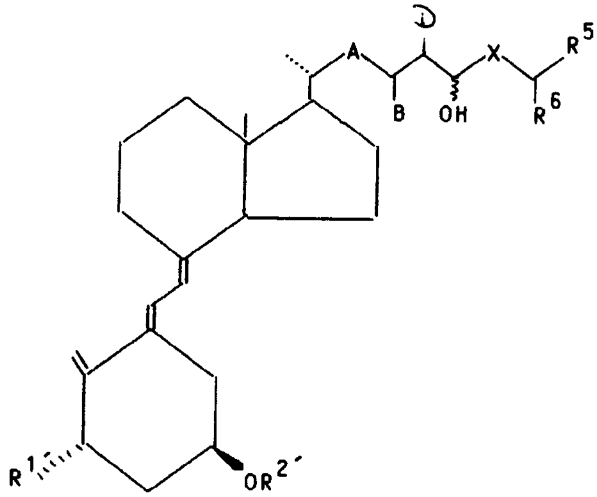

(IIIa) ~~~OH = α-OH
(IIIb) ~~~OH = β-OH

worin
$R^{1'}$, $R^{2'}$, A, X sowie $R^5$ und $R^6$ die in Formel IV und B und D die in Formel I angegebene Bedeutung haben, durch Bestrahlung mit ultraviolettem Licht unter Umkehr der Stereoisomerie an der 5,6-Doppelbindung in eine Verbindung der allgemeinen Formel II

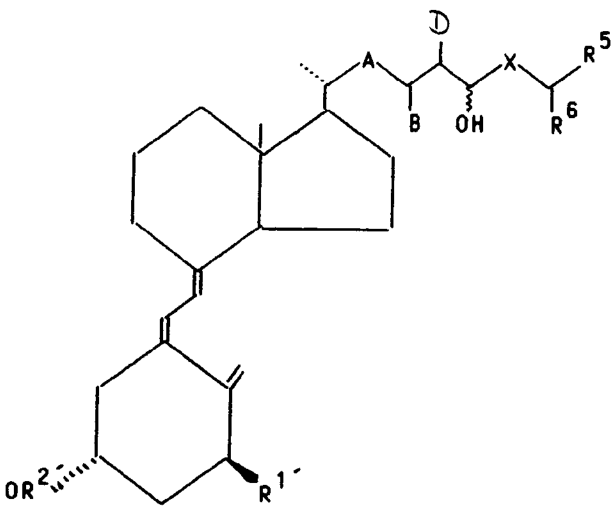

(II),

worin
$R^{1'}$, $R^{2'}$, A, B, D, X sowie $R^5$ und $R^6$ die in Formel IIIa/IIIb angegebene Bedeutung haben, umgewandelt
und diese anschließend durch Abspaltung vorhandener Hydroxyschutzgruppen und gegebenenfalls durch partielle oder vollständige Veresterung der Hydroxygruppen in eine Verbindung der allgemeinen Formel I überführt wird.

**10.** Pharmazeutische Präparate, dadurch gekennzeichnet, daß sie mindestens eine Verbindung gemäß den Ansprüchen 1 bis 8 sowie einen pharmazeutisch verträglichen Träger enthalten.

**11.** Verwendung der Verbindungen gemäß den Ansprüchen 1 bis 8 zur Herstellung von Arzneimitteln.

## Claims

**1.** Vitamin D derivatives with homologous side-chains in accordance with formula I

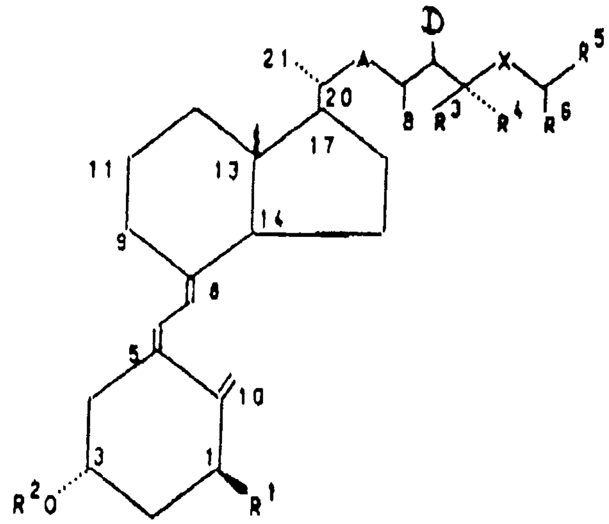

(I),

| | |
|---|---|
| where | |
| $R^1$ | is a hydrogen atom, a hydroxy or an acyloxy group with 1 to 9 carbon atoms, |
| $R^2$ | is a hydrogen atom or an acyl radical with 1 to 9 carbon atoms, |
| $R^3$ or $R^4$ | is a hydroxy or an acyloxy group with 1 to 9 carbon atoms with the other substituent, in each case, being a hydrogen atom, or $R^3$ and $R^4$ together represent an oxygen atom, |
| $R^5$ and $R^6$, | when independent of each other, are linear or branched alkyl radicals having up to 5 carbon atoms, or a trifluoromethyl group or, together, represent a saturated, unsaturated or aromatic carbocyclic ring formed with the tertiary carbon atom or, with the inclusion of 1 or 2 N, O or S atoms, a heterocyclic ring with 3, 4, 5 or 6 members, |
| B and D | are either one hydrogen atom each or, together, represent a second bond (E-configurated double bond), |
| | and either |
| A | represents a direct link between carbon atoms 20 and 22, and |
| X | is an alkyleneoxy radical $-(CH_2)_nO-$ with n = 1 to 3 |
| | or |
| A | is a methylene bridge ($-CH_2-$) between carbon atoms 20 and 22, and |
| X | represents an alkylene radical $-(CH_2)_n-$ or an alkyleneoxy radical $(CH_2)_nO-$ with n = 1 to 3, or, when A means a direct link and B and D together represent a second bond, |

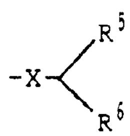

represents one of the radicals

$-CH_2-O-CH_2-$ 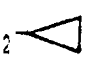 ,

$-(CH_2)_2- \equiv$ or $-(CH_2)_2- =$.

2. Vitamin D derivatives in accordance with Claim 1, where $R^1$ represents a hydroxy radical.

3. Vitamin D derivatives in accordance with Claim 1, where $R^2$ represents a hydrogen atom.

4. Vitamin D derivatives in accordance with Claim 1, where $R^3$ or $R^4$ represents a hydroxy radical.

5. Vitamin D derivatives in accordance with Claim 1, where n in X is 1 or 2.

6. Vitamin D derivatives in accordance with Claim 1, where $R^5$ and $R^6$ represent methyl groups.

7. Vitamin D derivatives in accordance with Claim 1, where $R^5$, $R^6$ and the tertiary carbon atom, together, represent a cyclopropyl ring.

8. 24-(1(R)-Hydroxy-4-methylpentyl)-9,10-secochola-5Z,7E,10(19), 23E-tetraene-1(S),3(R)-diol;
24-1(S)-hydroxy-4-methylpentyl)-9,10-secochola-5Z,7E,10(19),23E-tetraene-1(S),3(R)-diol;
24-(1(R)-hydroxy-3-methylbutyl)-9,10-secochola-5E,7E,10(19),23E-tetraene-1(S),3(R)-diol;
24-(1(S)-hydroxy-3-methylbutyl)-9,10-secochola-5Z,7E,10(19),23E-tetraene-1(S),3(R)-diol;
24-(1(R)-hydroxy-3-methylbuty1)-9,10-secochola-5Z,7E,10(19)-triene-1(S),3(R)-diol;
24-(1(S)-hydroxy-3-methylbutyl)-9,10-secochola-5Z,7E,10(19)-triene-1(S)-3(R)-diol;
24-(1(R)-hydroxy-3-isopropoxypropyl)-9,10-secocola-5Z,7E,10(19),23E-tetraene-1(S),3(R)-diol;
24-(1(S)-hydroxy-3-isopropoxypropyl)-9,10-secochola-5Z,7E,10(19),23E-tetraene-1(S),3(R)-diol;
24-isopropoxymethyl-9,10-secochola-5Z,7E,10(19),22E-tetraene-1(S),3(R)24(R)-triol;
24-isopropoxymethyl-9,10-secochola-5Z,7E,10(19),22E-tetraene-1(S),3(R),24(S)-triol;
24-(2-isopropoxyethyl)-9,10-secochola-5Z,7E,10(19),22E-tetraene-1(S),3(R),24(R)-triol;
24-(2-isopropoxyethyl)-9,10-secochola-5Z,7E,10(19),22E-tetraene-1(S),3(R),24(S)-triol;
26,27-cyclo-24a,24b-dihomo-9,10-secocholesta-5Z,7E,10(19),23E-tetraene-1(S),3(R)24a(R)-triol;
26,27-cyclo-24a,24b-dihomo-9,10-secocholesta-5Z,7e,10(19),23E-tetraene-1(S),3(R)24a(S)-triol.

9. Method for the manufacture of vitamin D derivatives with homologous side-chains in accordance with formula I

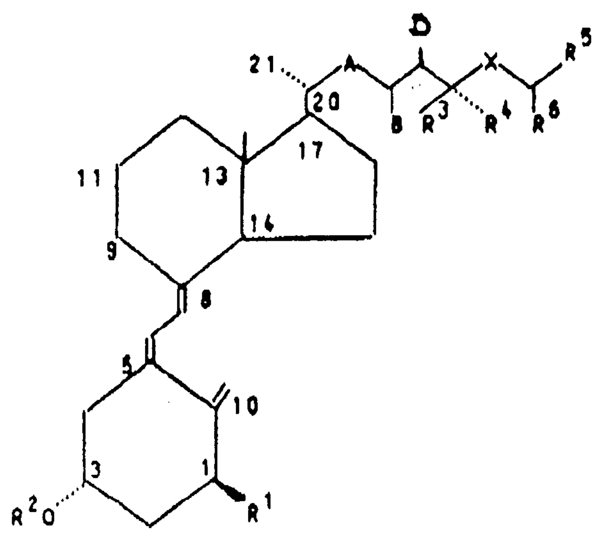

(I),

where $R^1$, $R^2$, $R^3$, $R^4$, $R^5$ and $R^6$ as well as A, B, D and X have the meaning indicated in Claim 1, characterized in that a compound of the general formula IV

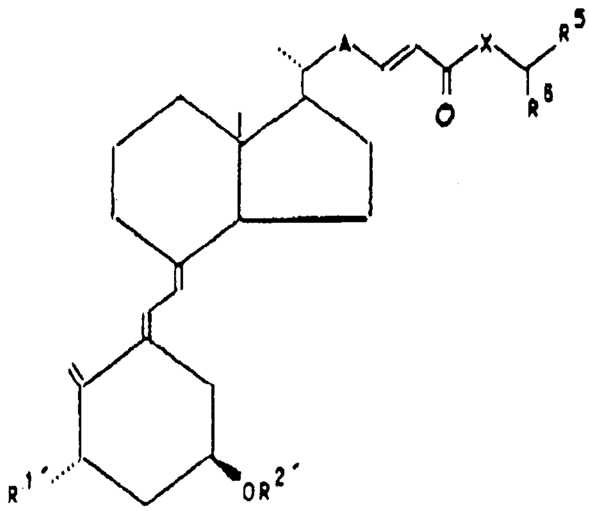

(IV),

where

| | |
|---|---|
| $R^{1'}$ | is a hydrogen atom or a protected hydroxy group, and |
| $R^{2'}$ | is a hydroxy protecting group, and |
| A, X as well as $R^5$ and $R^6$ | have the meaning indicated in formula I, |

is converted, if required after selective hydration of the side-chain double bond, into a compound of the general formula IVa

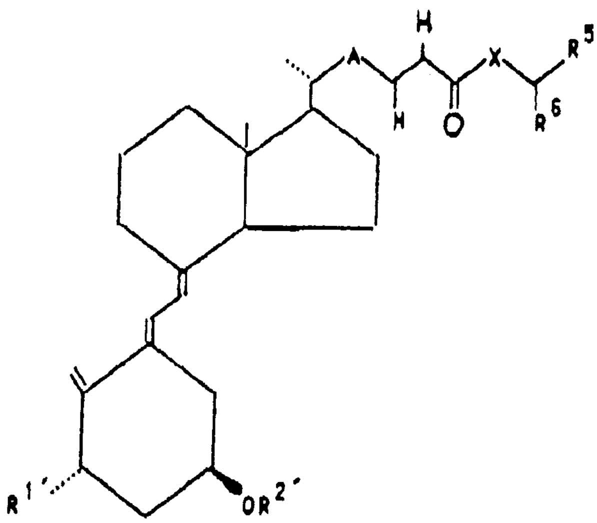

(IVa),

where

$R^{1'}$, $R^{2'}$, A, X as well as $R^5$ and $R^6$ have the meaning indicated in formula IV,

and is converted, if required after reduction of the carbonyl function, and possibly after separation of the mixture of epimeric hydroxy compounds formed by the reduction and represented by the general formulas IIIa and IIIb

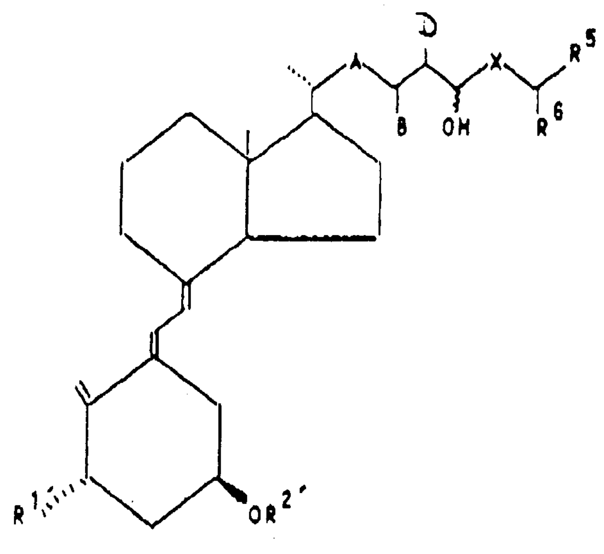

(IIIa) ~OH = α-OH
(IIIb) ~OH = β-OH

where

R1', R2', A, X as well as $R^5$ and $R^6$ have the meaning indicated in formula IV and B and D the meaning indicated in formula I

into a compound of the general formula II by irradiation with ultraviolet light under inversion of the stereoisomerism of the 5,6 double bond

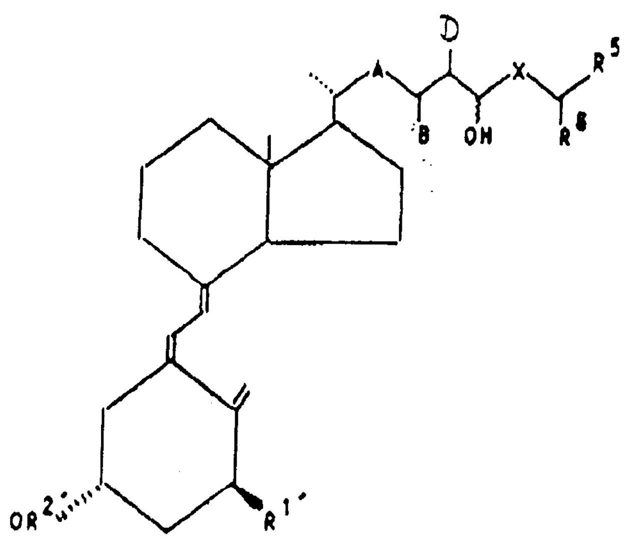

(II),

where

R1', R2', A, B, D, X as well as $R^5$ and $R^6$ have the meaning indicated in formula IIIa/IIIb,

and in that the latter is then converted into a compound of the general formula I by splitting off any hydroxy protecting groups present and, if necessary, partial or complete esterification of the hydroxy groups.

**10.** Pharmaceutical specialties, characterized in that they contain at least one compound in accordance with Claims 1 to 8 as well as one pharmaceutically tolerated carrier.

**11.** Use of the compounds in accordance with Claims 1 to 8 in the manufacture of drugs.

## Revendications

**1.** Dérivés de la vitamine D à chaînes latérales homologues suivant la formule I

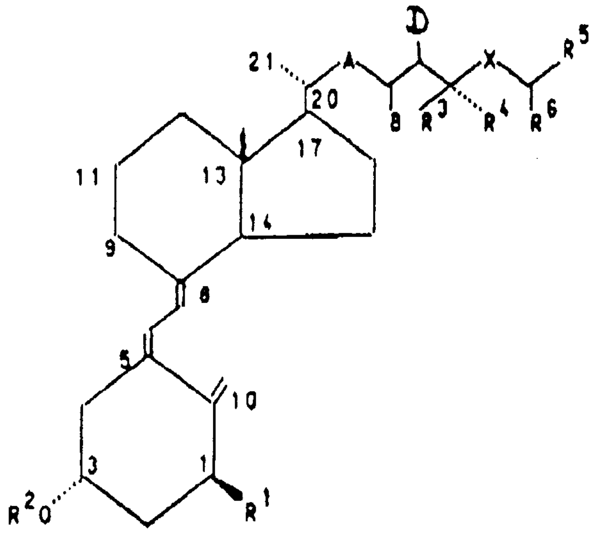

(I),

où

$R^1$ est un atome d'hydrogène, un hydroxyle ou un groupe acyloxy possédant 1 à 9 atomes de carbone,

$R^2$ est un atome d'hydrogène ou un groupe acyle possédant 1 à 9 atomes de carbone,

$R^3$ ou $R^4$ est un hydroxyle ou un groupe acyloxy possédant 1 à 9 atomes de carbone, l'autre substituant étant un atome d'hydrogène, ou $R^3$ et $R^4$ ensemble représentent un atome d'oxygène,

$R^5$ et $R^6$, où ils sont indépendents, représentent chacun un radical d'alkyle linéaire ou ramifié possédant jusqu'à 5 atomes de carbone, ou un groupe trifluorométhyle ou, ensemble, un composé carbocyclique saturé, non saturé ou aromatique formé conjointement avec l'atome de carbone tertiaire ou, par inclusion de 1 ou 2 atomes N, O ou S, un composé hétérocyclique à 3, 4, 5 ou 6 chaînons,

B et D, où ils sont indépendents, sont chacun un atome d'hydrogène ou, ensemble, représentent une deuxième liaison (double liaison en configuration E), et

ou bien

A est une liaison directe entre les atomes de carbone 20 et 22, et

X est un radical alkylèneoxy $-(CH_2)_nO-$ où n = 1 à 3,

ou bien

A est un pont methylène ($-CH_2-$) entre les atomes de carbone 20 et 22, et

X représente un radical alkylène $-(CH_2)_n-$ ou un radical alkyléneoxy $-(CH_2)_nO-$ où n = 1 à 3,

ou, dans les cas où A est une liaison directe et B et D ensemble représentent une deuxième liaison,

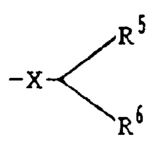

représente un des radicaux

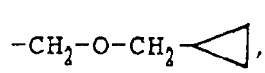

$-(CH_2)_2- \equiv$ ou $-(CH_2)_2- =$.

2. Dérivés de la vitamine D suivant la revendication 1, où $R^1$ représente un hydroxyle.

3. Dérivés de la vitamine D suivant la revendication 1, où $R^1$ représente un atome d'hydrogène.

4. Dérivés de la vitamine D suivant la revendication 1, où $R^3$ ou $R^4$ représente un hydroxyle.

5. Dérivés de la vitamine D suivant la revendication 1, où n dans X est 1 ou 2.

6. Dérivés de la vitamine D suivant la revendication 1, où $R^5$ et $R^6$ représentent des groupes méthyle.

7. Dérivés de la vitamine D suivant la revendication 1, où $R^5$, $R^6$ et l'atome de carbone tertiaire représentent conjointement un composé cycloprppyle.

8. 24-(1(R)-hydroxy-4-méthylpentyl)-9,10-sécochola-52,7E,10(19),23E-tetraène-1(S),3(R)-diol;
24-(1(S)-hydroxy-4-méthylpentyl)-9,10-sécochola-5Z,7E,10(19),23E-tetraène-1(S),3(R)-diol;
24-(1(R)-hydroxy-3-méthylbutyl)-9,10-sécochola-5Z,7E,10(19),23E-tetraène-1(S),3(R)-diol;
24-(1(S)-hydroxy-3-méthylbutyl)-9,10-sécochola-5Z,7E,10(19),23E-tetraène-1(S),3(R)-diol;
24-(1(R)-hydroxy-3-méthylbutyl)-9,10-sécochola-5Z,7E,10(19)-triène-1(S),3(R)-diol;
24-(1(S)-hydroxy-3-méthylbutyl)-9,10-sécochola-5Z,7E,10(19)-triène-1(S)-3(R)-diol;
24-(1(R)-hydroxy-3-isopropoxypropyl)-9,10-sécocola-5Z,7E,10(19),23E-tetraène-1(S),3(R)-diol;
24-(1(S)-hydroxy-3-isopropoxypropyl)-9,10-sécochola-5Z,7E,10(19),23E-tetraène-1(S),3(R)-diol;
24-isopropoxyméthyl-9,10-sécochola-5Z,7E,10(19),22E-tetraène-1(S),3(R)24(R)-triol;
24-isopropoxyméthyl-9,10-sécochola-5Z,7E,10(19),22E-tetraène-1(S),3(R),24(S)-triol;
24-(2-isopropoxyéthyl)-9,10-sécochola-5Z,7E,10(19),22E-tetraène-1(S),3(R),24(R)-triol;
24-(2-isopropoxyéthyl)-9,10-sécochola-5Z,7E,10(19),22E-tetraène-1(S),3(R),24(S)-triol;
26,27-cyclo-24a,24b-dihomo-9,10-sécocholesta-5Z,7E,10(19),-23E-tetraène-1(S),3(R)24a(R)-triol;
26,27-cyclo-24a,24b-dihomo-9,10-sécocholesta-5Z,7e,10(19),-23E-tetraène-1(S),3(R)24a(S)-triol.

9. Procédé pour la fabrication de dérivés de la vitamine D à chaînes latérales homologues suivant la formule I

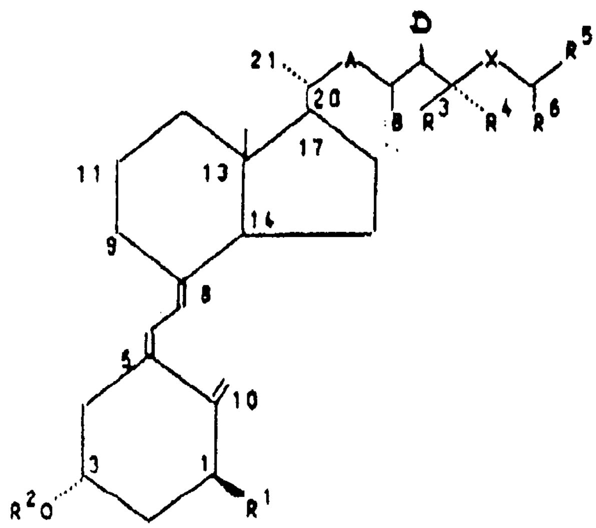

(I),

où $R^1$, $R^2$, $R^3$, $R^4$, $R^5$ et $R^6$ ainsi que A, B, D et X ont la signification indiquée dans la revendication 1, caractérisé en ce qu'un composé de la formule générale IV

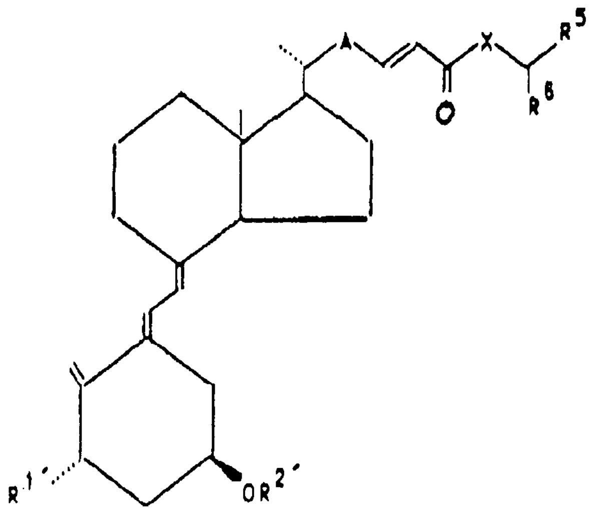

(IV),

où

R1' est un atome d'hydrogène ou un hydroxyle protégé et

R2' est un groupe hydroxy de protection et

A, X ainsi que $R^5$ et $R^6$ ont la signification indiqué dans la formule I,

est transformé, le cas échéant après hydratation sélective de la liaison double dans la chaîne latérale, en un composé de la formule générale IVa

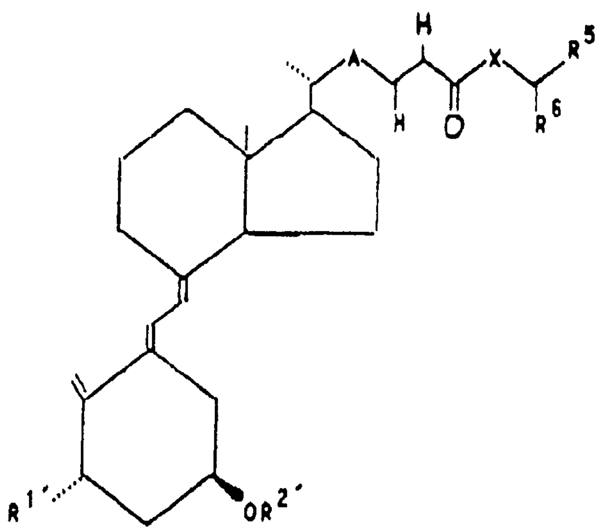

(IVa),

où

R1', R2', A, X ainsi que $R^5$ et $R^6$ ont la signification indiquée dans la formule IV,

et est transformé, le cas échéant après réduction de la fonction carbonyle et, le cas échéant, après séparation du mélange des composés épimères hydroxy des formules générales IIIa et IIIb formés par la réduction

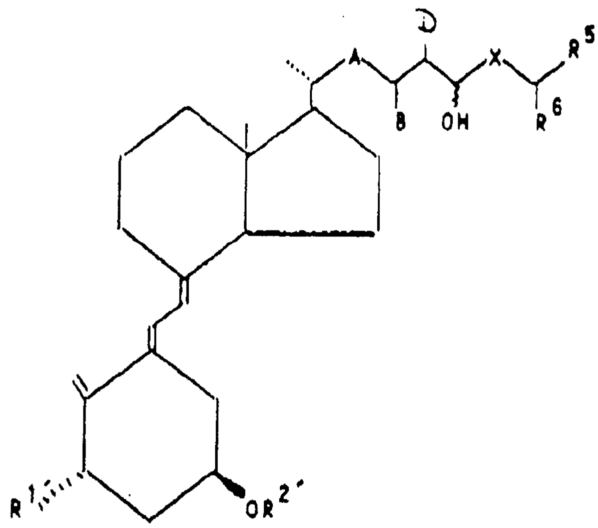

(IIIa) ~~OH = α-OH
(IIIb) ~~OH = β-OH

où

R1', R2', A, X ainsi que $R^5$ et $R^6$ ont la signification indiquée dans la formule IV et B et D ont la signification indiquée dans la formule I

par exposition à la lumière ultraviolette et sous inversion de la stéréoisomérie dé la liaison double 5,6 en un composé de la formule générale II

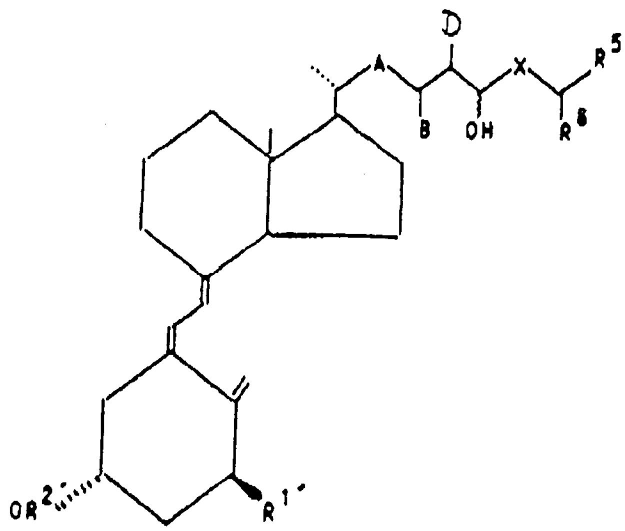

(II),

où

R1', R2', A, B, D, X ainsi que $R^5$ et $R^6$ ont la signification indiquée dans la formule IIIa/IIIb,

et que ce composé est ensuite transformé par élimination de groupes hydroxy de protection existants et, le cas échéant par estérification partielle ou complète des groupes hydroxy, en un composé de la formule générale I.

**10.** Préparations pharmaceutiques, caractérisées en ce qu'elles contiennent au moins un des composés suivant les revendications 1 à 8 ainsi qu'un véhicule pharmaceutiquement tolérable.

**11.** Utilisation des composés suivant les revendications 1 à 8 dans la fabrication de médicaments.